(19) Europäisches Patentamt
European Patent Office
Office européen des brevets

(11) EP 2 263 649 A1

(12) **EUROPEAN PATENT APPLICATION**
published in accordance with Art. 153(4) EPC

(43) Date of publication:
**22.12.2010 Bulletin 2010/51**

(21) Application number: **09713580.0**

(22) Date of filing: **16.02.2009**

(51) Int Cl.:
***A61K 9/08*** *(2006.01)* ***A61K 33/24*** *(2006.01)*
***A61K 31/198*** *(2006.01)* ***A61K 38/00*** *(2006.01)*
***A61P 37/00*** *(2006.01)* ***A61P 43/00*** *(2006.01)*

(86) International application number:
**PCT/RU2009/000070**

(87) International publication number:
**WO 2009/104988 (27.08.2009 Gazette 2009/35)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **21.02.2008 RU 2008106419**

(71) Applicant: **"Ivy Pharm" Jsc.**
**St.Petersburg 190121 (RU)**

(72) Inventors:
- **BALAZOVSKY, Mark Borisovich**
  **St.Petersburg 199106 (RU)**
- **ANTONOV, Viktor Georgievich**
  **Leninskaya obl. 188643 (RU)**
- **BELYAEV, Alexandr Nikolaevich**
  **St.Petersburg 192212 (RU)**
- **EREMIN, Alexei Vladimirovich**
  **St.Petersburg 198260 (RU)**

(74) Representative: **Bradley, Adrian et al**
**Cleveland**
**40-43 Chancery Lane**
**GB-London WC2A 1JQ (GB)**

(54) **PHARMACOLOGICAL ADJUVANTS BASED ON COORDINATION COMPOUNDS OF D-METALS**

(57) The invention relates to the use of small and ultra small quantities of bi- and oligo-nuclear forms of the coordination compounds of d-metals in the form of drugs or agents for enhancing the therapeutic efficiency of pharmacological substances.

EP 2 263 649 A1

Printed by Jouve, 75001 PARIS (FR)

## Description

Field of industry

The present invention relates to the field of the production of medicinal products, and can be employed in pharmacology, human medicine and veterinary medicine.

Prior art

The reality of modern medical practice is increasing prevalence of chronic infectious and noninfectious proliferative-inflammatory diseases, characterized by a continuously relapsing course, multidrug resistance, and consequently inadequate efficacy of medicinal products for etiotropic and pathogenetic therapy. Chronic infectious and noninfectious proliferative-inflammatory diseases are one of the commonest causes of hospitalization and disablement of the population in the age range from 20 to 60 years. The predominant approach to therapy of chronic diseases of varying etiology, directed at the use of biologically highly active compounds, expansion of the range of pharmacotherapeutic agents that can be used, and increase in the therapeutic dose of pharmaceutical preparations, predetermined the manifestation of the most serious problem in drug therapy, defined as medication-induced death, which is now in fifth place among the causes of patient death [8].

Improving the pharmacokinetics and/or pharmacodynamics of existing medicinal products is an alternative to increasing the dosage for overcoming resistance to a medicinal product. Thus, several combination drugs are known, including co-amoxiclav, composed of amoxicillin and clavulanic acid, and Tienam, containing imipenem in combination with a specific inhibitor of the kidney enzyme dihydropeptidase - cilastatin. Clavulanic acid prevents decomposition of amoxicillin by bacterial enzymes, and cilastatin inhibits metabolism of imipenem in the kidneys, which greatly increases the concentration of unchanged antibiotic in the kidneys and urinary tract [4].

However, an enormous number of other medicinal products exist, that are potentially useful for the treatment of diseases, but do not provide the desired effect owing to the development of one or other kind of resistance to them.

For therapy, it is also useful to additionally prescribe general restoratives and immunostimulants.

However, in clinical practice the use of agents for replacement therapy and/or with stimulating effects on cellular and/or humoral components of the immune system, the functional activity of the cells of the liver, kidneys, and other organs in conditions of a physiologically inadequate and/or insufficient level of intercellular, humoral-cellular and matrix-cellular interactions, and reduced capacities of hepatocytes and other cells for neutralizing toxic substances is accompanied by a range of complications, in severity comparable to or exceeding the underlying disease.

At the present time, a substance is known, which in itself possesses remarkably varied pharmacological activity and low toxicity, and moreover has been found to increase the therapeutic efficacy of a wide range of medicinal products. This substance is glycyrrhizic acid, which is a metabolite of the root of root licorice and Russian licorice *(Glycyrrhiza glabra L., Gl. uralensis)* and derivatives thereof [2]. Derivatives of glycyrrhizic acid form clathrates with pharmaceutical substances in situ when administered simultaneously. It was found that formation of a clathrate of a pharmaceutical substance with a derivative of glycyrrhizic acid has the result that these pharmaceutical substances display a therapeutic effect at reduced doses, which offers the prospect of lowering the dosage of many medicinal products.

However, the problem of producing low-dose pharmaceutical preparations is still urgent. Furthermore, the solution described above does not make it possible to overcome development of drug resistance.

Disclosure of the invention

Thus, the object of the present invention is to create an agent that would above all make it possible to restore physiologically adequate reactions of the organism, which should promote an increase in efficacy of endogenous substances, and that would also make it possible to increase the therapeutic efficacy of drugs administered to the patient, as well as make it possible to reduce the dose and/or overcome drug resistance.

This object is achieved in that compositions are proposed that contain, in aqueous solution, bi- or oligonuclear coordination compounds, formed by identical or different atoms of d-metals, joined together by one or more bridging groups and containing, in the composition of the inner sphere of each d-metal atom, water molecules and/or hydroxide groups, as well as labile ligands, which are easily replaced with the sulfur atoms of thioamino acids or peptides, such as mono-, bi- or higher-dentate oxygen-containing and/or nitrogen-containing and/or sulfur-containing organic molecules with bridging and nonbridging types of coordination to atoms of d-metals.

In the proposed compositions the composition of the inner sphere of each d-metal atom can also include strongly bound ligands of any nature and coordination number with bridging and nonbridging types of coordination to atoms of d-metals.

The proposed composition can also comprise free molecules of said labile ligand, not coordinated to d-metal

atoms.

It is desirable for the concentration of each d-metal or the total concentration of several d-metals in said aqueous solution to be less than $10^{-3}$ M.

It is preferable if the molar ratio of d-metal atom to molecule of labile ligand is from 1:1 to 1:30000.

This coordination compound can be obtained by interaction of aqua-hydroxo complexes of d-metals and solutions of the aforesaid labile ligands.

The proposed composition has an effect on the biological activity of cells by modulating the activity of extracellular, cell-surface and intracellular receptors, enzymes, ion channels, proteins of transporters of the cytoplasmic and intracellular membranes, extracellular regulatory and transport molecules of a peptide nature. It possesses specific cellular tropism and/or organotropism and/or specific systemic action and at least one pharmacological activity, selected from the group comprising antiviral, antibacterial, antiproliferative, proapoptotic, cytoprotective, direct and indirect antioxidant, hemostimulating, antihypoxanthine, immunomodulating, toxicity-modifying, antifibrotic, antiinflammatory activity, as well as the capacity to suppress reactions of multiple drug resistance of various genesis, to modulate regulatory, metabolic, and immune dysfunctions and imbalances, which can be used for production of a medicinal product for external, inhalational, enteral and parenteral application.

The proposed composition is able to increase the therapeutic efficacy of pharmacologically active substances.

It is desirable if the pharmacologically active substance, whose therapeutic efficacy is increased, and the aforesaid labile ligand are one and the same substance.

However, an embodiment of the invention is possible in which this pharmacologically active substance and the aforesaid labile ligand are different substances.

When the pharmacologically active substance and the labile ligand are different substances, the proposed composition according to the invention can comprise free molecules of said pharmacologically active substance, as well as, possibly, free molecules of the labile ligand.

It is desirable if the pharmacodynamics and/or pharmacokinetics of the coordination compound correspond to that of said pharmacologically active substance.

The transition metal in the composition of the coordination compound is iron, copper, palladium or some other d-metal.

The proposed composition can comprise coordination compounds of different d-metals.

The composition according to the invention can be in liquid or dried form, including lyophilized form. Prior to administration, said dried form may be reconstituted with water of acceptable quality.

A method of therapeutic action on a patient's body is also proposed, in which a patient requiring this is administered an effective amount of the composition according to the invention.

A method is also proposed for increasing the therapeutic efficacy of a pharmacologically active substance, in which the patient is administered the composition according to the invention simultaneously or successively with said substance.

The aforesaid pharmacologically active substance and the composition according to the invention can be administered in a composition of a single dosage form, or in a composition of different dosage forms, and then they are administered either simultaneously or successively. Increase in therapeutic efficacy of the pharmacologically active substance can be achieved with low or ultra-low concentrations of the composition according to the invention.

In the proposed methods it is desirable if the amount of the coordination compound of a d-metal or the total concentration of coordination compounds of several d-metals administered to the patient is less than $10^{-3}$ mol/kg of body weight. It is preferable if the amount of d-metal administered or the amount of each d-metal in combined use in the composition of coordination compound does not exceed the physiologically permissible values for said metal. However, this value may be exceeded when large amounts of d-metal or of several d-metals administered in the composition of coordination compound are required for achieving a therapeutic effect.

In these methods, a patient requiring this is administered an effective amount of the composition according to the invention as drops intranasally or orally, by inhalation, enterally, parenterally or by external application.

The composition according to the invention restores adequate response of the organism both to endogenous, i.e. synthesized within the organism, and to pharmacologically active substances introduced from outside of the organism. It is probable that the active conformation of the target molecules is restored and oxidative-reducing (redox) regulation of the whole system is provided. Apparently, this takes place because the coordination compound of d-metal in the composition according to the invention is able to provide catalysis of the redox reaction, when the free thiol groups of cysteines are oxidized with formation of disulfide bonds, and the disulfide bonds are reduced with formation of free thiol groups.

When co-administered with any medicinal product, the coordination compound increases the sensitivity of the primary target to the medicinal product and/or lowers the toxicity of the medicinal product or products of metabolism thereof by inducing enzymes of the second phase of detoxication of xenobiotics.

Brief description of the drawings

Fig. 1. Binuclear metal frameworks.
Fig. 2. Oligonuclear metal frameworks.
Fig. 3. Influence of cysteine on the tyrosine kinase activity of the epidermal growth factor receptor (1). Influence of a coordination compound of cysteine and copper on the tyrosine kinase activity of the epidermal growth factor receptor (2). Combined effect of cysteine and a coordination compound of cysteine and copper, used in the ratio of ligand to coordination compound of 1000:1, on the tyrosine kinase activity of the epidermal growth factor receptor (3).
Fig. 4. Tyrosine kinase activity of the epidermal growth factor receptor when glycine (1), coordination compound of glycine and copper (2), and glycine in combination with the coordination compound in the ratio 1000:1 (3) are added to the incubating medium.
Fig. 5. Nature of the response of MAP kinases ERK1,2 (p ERK) when cysteine (1), coordination compound of cysteine and copper (2) and cysteine in combination with the coordination compound in the ratio 1000:1 (3) are added to the cell incubating medium.
Fig. 6. Nature of the response of MAP kinases ERK1,2 (p ERK) when glycine (1), coordination compound of glycine and copper (2) and glycine in combination with the coordination compound in the ratio 1000:1 (3) are added to the cell incubating medium.
Fig. 7. Nature of expression of hemoxygenase 1 when cysteine (1), coordination compound of cysteine and copper (2) and cysteine in combination with the coordination compound in the ratio 1000:1 (3), act upon cells.
Fig. 8. $Ca^{2+}$ signals induced by ATP (1, 3) and thapsigargin (2, 4) in peritoneal macrophages in a medium containing $Ca^{2+}$ ions (1, 2) and in a calcium-free medium (3, 4).
Fig. 9 shows the influence of cysteine on $[Ca^{2+}]_i$ at rest and $Ca^{2+}$ signals induced by 200 μM ATP (1, 2) and 0.5 μM thapsigargin (3) in macrophages, in normal physiological solution (a) or in nominally calcium-free medium (2, 3).
Fig. 10. Influence of a coordination compound (CC) of palladium bound to cysteine on the intracellular calcium concentration ($[Ca^{2+}]_i$) at rest and $Ca^{2+}$ signals due to ATP. The coordination compound cancels the inhibitory effect of the selective calcium channel inhibitor nifedipine (1), and the effect of the coordination compound itself is suppressed by the reducing agent dithiothreitol (DTT) (2).
Fig. 11. Flowchart of pharmacological decisions on the use of small and ultra-small amounts of coordination compounds of d-metals for production of novel medicinal products.

1 - medicinal products based on coordination compounds - pharmacological decisions on application of the biological activity of coordination compounds and the corresponding pharmacological effects.
2 - low-dose medicinal products based on coordination compounds in combination with combinable pharmacological substances of pharmacopeial medicinal products - pharmacological decisions on intensification of pharmacological activity and/or reduction of toxicity of the active principle of pharmacopeial medicinal products.

Carrying out the invention

In this description, with reference to the present invention the terms "complex compound" and "coordination compound" are synonymous and are used with their conventional meaning, i.e. they are to be understood as compounds containing a group of ions or neutral molecules, called ligands, arranged (coordinated) in a defined order around a central atom (ion), called a complexing agent. Coordination compounds are compounds existing both in the crystalline state, and in solution, characterized by the presence of a central atom (acceptor of unshared electron pairs), surrounded by ligands (donors of unshared electron pairs). The ligands are able to split off in stages and reversibly from the central atom according to a heterolytic type. In the majority of cases, in molecular form, coordination compounds can be regarded as consisting of simple molecules, capable of independent existence.

It should be pointed out that the term "complex compound" is sometimes used in another sense, e.g. with reference to molecular complexes, in which it is not possible to indicate a coordination center, as well as inclusion compounds. The ligands in such compounds can be ions or molecules of an inorganic, organic or organoelemental nature. These ligands either are not bound to one another or they mutually repel, or they are joined by forces of intermolecular attraction such as van der Waals interactions. Examples of "complex compounds" in the broader sense of this word are clathrates, formed by glycyrrhizic acid. These clathrates, however, are not included among the coordination compounds. The complexing agent-ligands system is called the "inner coordination sphere" (in the nomenclature it is usually separated by square brackets from the "outer coordination sphere"). The outer coordination sphere is made up of a certain number of oppositely charged particles (and sometimes an additional number of solvate molecules).

Coordination compounds whose molecules have several metal atoms, surrounded by ligands and joined to one another by means of bridging groups, are called polynuclear (multinuclear), and those with two atoms are called binuclear.

The coordination number of a ligand means the number of bonds between the ligand and the central atom - the complexing agent.

In this description the terms "d-metals", "transition metals" and "transition element" are identical, and refer to the chemical elements of the periodic system, in which there is filling of d-sublevels with electrons. These metals are located in supplementary subgroups of groups I-VIII of Mendeleev's periodic table of the elements (groups 3-12 in the long-period variant of the table).

The d-metals include, in particular, Fe, Co, Zn, Cu, V, Cr, Mn, Ni, and Mo. These metals are also called d-biometals (essential for life), since these d-metals are included in the composition of biosystems.

According to the present invention, the presence of two or more coordination centers in the composition of a coordination compound, as well as the presence of water molecules coordinated to the d-metal, the presence of oxo/hydroxo groups in the composition of a coordination compound, and the presence of labile ligands replacing the sulfur atoms of amino acids or peptides, are necessary conditions, which allow said compound (composition) to display its pharmacological action.

A great many reactions are now known in which coordination compounds of transition elements act as catalysts of the oxidation of organic compounds. Redox catalytic behavior is mostly characteristic of transition metals, mainly such as Mn, Fe, Co, Cu, V etc., since their main characteristic feature is the capacity of the ion of said metal to exist in solution in more than one oxidation state [1].

It is a well established fact that a feature of the hydrolysis of many coordination compounds and aquated transition metal ions is a process leading to formation of oligonuclear compounds with bridging hydroxo groups, via which effective electron exchange can take place between the metal atoms [1].

The present inventors have discovered special properties of multinuclear aqua/hydroxo complexes of d-metals. Said complexes provide catalysis of a redox reaction involving sulfur, as a result of which sulfur, which is included in the composition of amino acids, and in particular cysteines, can be mildly oxidized with formation only of disulfide bonds (crosslinks), which promote restoration of the active conformation of the molecules of receptors. Complexes of the same d-metals, taken in unhydrolyzed form, when used as catalysts can lead to deeper oxidation of sulfur, even to the formation of taurine from cysteine, which is unfavorable, since this modification in a biological system is irreversible and is negative for the functional activity of biomolecules. Thus, the multinuclear aqua/hydroxo complexes of d-metals according to the invention ensure that the coordination compound displays the desired therapeutic properties (in particular restoration of adequate physiological response and redox regulation).

It should be pointed out that for oligomerization to occur, with formation of multinuclear complexes, the solution of the d-metal compound must be diluted to a sufficient degree. That is, dissolution of the d-metal compound in water certainly does not mean formation of bi- or oligo-nuclear aqua/hydroxo complexes in the solution. Only sufficiently diluted solutions provide the desired structure of the complex. Attention should also be drawn to the fact that the process of formation of aqua/hydroxo complexes in solution is of a reversible character. However, introduction of a labile ligand predetermines the formation of bi- and oligonuclear complexes of d-elements and shifts the equilibrium of the reaction toward formation of a complex whose structure includes an organic ligand, coordinated with a d-metal associated with aqua/hydroxo groups.

"Aqua complex" means a coordination compound containing water molecules (one or more) as ligands. "Hydroxo complex" means a coordination compound containing one or more hydroxide ($OH^-$) groups as ligands. "Aqua-hydroxo complex" means a coordination compound containing one or more hydroxide ($OH^-$) groups and water molecule (s) as ligands.

Typical metal frameworks of the coordination compound in a composition according to the invention are shown in Fig. 1 and Fig. 2.

It should be pointed out that the ligand in the coordination compound according to the invention must be labile, i.e. it must easily be replaced by sulfur atoms in the composition of amino acids, oligo- and polypeptides and proteins.

Oxygen-containing, nitrogen-containing or sulfur-containing compound, as used in this description, denotes any compound in which there is an atom of oxygen, nitrogen or sulfur, via which said compound can be coordinated to a metal atom. That is, in a compound according to the invention, coordination of the ligand is via an oxygen atom, sulfur atom or nitrogen atom. In a polynuclear complex said ligand can easily be displaced by other ligands, which can serve as substrates of the oxidation reaction. Coordination compounds according to the invention can also additionally contain strongly bound ligands of any nature and coordination number with bridging and nonbridging types of coordination to atoms of d-metals. Said ligands can be organic and inorganic ligands, containing donor atoms of p-elements, which are not replaced by sulfur atoms of thioamino acids or peptides. Said ligands can endow the coordination compound according to the invention with high thermodynamic and kinetic stability.

In this description the term "ligand" will further denote in fact a labile, rather than a strongly bound ligand, unless

the opposite is stated directly.

Examples of labile ligands according to the present invention are amino acids, peptides (including di-, tri-, tetra-, penta- and hexapeptide), purine or pyrimidine bases, and aminothiols. Concrete examples of amino acids are cysteine and glycine. Other possible ligands are mercaptoethylamine, pyrimidine etc.

The composition according to the invention can be obtained in any way, which will be clear to a person skilled in the art, on the basis of the proposed structure of the coordination compound.

The starting compound used for production of the composition and coordination compound according to the invention can be any d-metal compound, in particular a salt or a coordination compound, for example: $Fe_2SO_4$, $FeSO_4 \cdot 7H_2O$, $FeCl_3 \cdot 6H_2O$, $(NH_4)_2Fe(SO_4)_2 \cdot 6H_2O$, $(NH_4)Fe(SO_4)_2 \cdot 12H_2O$, $Cu_2(\mu\text{-}O_2CCH_3)_4(H_2O)_2)$, $CuCl_2 \cdot 2H_2O$, $[Pd_2(\mu OH)_2(NH_3)_4]Cl_2$, $(NH_4)MoO_4$, $Rh_2(\mu\text{-}O_2CCH_3)_4(H_2O)_2$, $[Co(NH_3)_6]Cl_3$, $[Pd_2(\mu\text{-}Cys)_2(dipy)_2](NO_3)_2$ etc.

For production of the coordination compound according to the invention it is possible to use an aqueous solution of any d-metal compound and add to it a labile ligand in dry form or in the form of aqueous solution. Formation of polynuclear aqua/hydroxo complexes takes place in the solution obtained.

As an alternative, a greatly diluted aqueous solution of any d-metal compound, in which formation of polynuclear aqua/hydroxo complexes of a d-metal has already taken place, can be obtained first, and then the ligand in dry form or in the form of aqueous solution can be added to it. The degree of dilution for each actual compound can be selected empirically. A precise judgment about formation of polynuclear aqua/hydroxo complexes can be based on physicochemical investigations (in particular spectral, and in some cases X-ray diffraction investigations) and the results of modern quantum-chemical calculations confirming them [10, 11]. An indirect criterion of the presence of such a structure is catalysis *in vitro* of a redox reaction by said compound, where the sulfur of thio groups is oxidized with formation of only disulfide bonds.

Any molar ratios of aqua-hydroxo complex to ligand are possible in principle, but ratios from 1:1 to 1:30000 are desirable. A ratio of 1:1 is a sufficient condition for formation of the catalyst (one ligand molecule to one polynuclear aqua-hydroxo complex). In cases when it is desirable for the ligand to occupy more than one coordination site in the complex, the proportion of ligand is increased accordingly.

The coordination compound according to the invention can be formed both in non-oxidative and in oxidative conditions, and this does not affect its activity. If interaction of the aqua-hydroxo complex with the ligand takes place in oxidative conditions, the ligand can perform the role of substrate for the catalyst in the oxidation reaction, and this does not prevent it from carrying out its therapeutic function.

The solution obtained can be dried and can be stored in dry form until it is used. In the transition "greatly diluted solution of complex of d-element - solid phase of complex" the structure of the complex undergoes a change, but it is restored on subsequent dilution with water. Subsequent restoration with water leads to production of a compound with identical activity.

The present inventors proposed one of the methods of production of a coordination compound of a d-metal, in which a solution of the d-metal compound is added to a solution of the ligand, the ligand being used in excess. This means that the amount of ligand that is bound is more than is necessary for formation of the coordination compound with the d-metal. For a range of concentrations with respect to the d-metal in the composition of the coordination compound $10^{-3}$ - $10^{-12}$ M, the excess of ligand can be a multiple of 10-30000 units.

Preliminary dilution of the salt of d-metal or d-metals, and of the ligand leads to production of a solution of coordination compounds of homogeneous composition.

The proposed method, in which, for formation of the coordination compound, the ligand is used in excess with respect to the d-metal, offers the possibility of working with small amounts of coordination compounds.

One of the important characteristics of the catalyst obtained is its stability, i.e. the number of catalytic cycles the catalyst can withstand. The catalysts obtained according to the proposed method display very high stability.

Coordination compounds of d-metals according to the present invention are used in the form of pharmaceutical preparations (dosage forms). Pharmaceutical preparations of coordination compounds of d-metals can be administered orally, i.e. dosage forms such as solutions, emulsions or suspensions, as well as tablets, film-coated tablets, sugar-coated pills, hard and soft gelatin capsules. Rectal administration may also be effective, i.e. dosage forms such as suppositories. Parenteral administration is effective, i.e. forms of solutions for subcutaneous, intramuscular, intravascular, intracavitary injections. The composition according to the invention can also be used externally (ointments, creams etc.) or can be administered by inhalation, as drops, intranasally and/or orally.

For production of pharmaceutical preparations (dosage forms), therapeutically inert, inorganic or organic vehicles are added to the coordination compounds of d-metals. Lactose, maize starch or its derivatives, talc, stearic acid or its salts etc. can be used, for example, as said vehicles for tablets, film-coated tablets, sugar-coated pills and hard gelatin capsules. Suitable vehicles for soft gelatin capsules are, for example, vegetable oils, waxes, fats, semisolid and liquid polyols etc. Suitable vehicles for production of solutions and syrups are, for example, water, polyols, sucrose, invert sugar, glucose etc. Suitable vehicles for suppositories are, for example, natural or solidified oils, waxes, fats, semiliquid or liquid polyols etc.

In addition, pharmaceutical preparations can contain preservatives, solubilizers, stabilizers, wetting agents, emulsifiers, sweeteners, colorants, correctants, salts for controlling osmotic pressure, buffers, masking agents or antioxidants and other components necessary for preparation of the dosage form.

The aforementioned auxiliary substances, used for formation of dosage forms, will be called hereinafter "therapeutically inert excipients". However, the dosage forms of the composition according to the invention can also contain other therapeutically active substances.

According to the invention, the proposed composition can be used for intensifying the therapeutic activity of a pharmacologically active substance.

In the context of this description, increase in therapeutic efficacy of a pharmacologically active substance means a decrease in single dose or total dose or a decrease in toxicity at the accepted therapeutic dose of said pharmacologically active substance.

"Pharmacologically active substance" means any substance that is used for therapeutic purposes.

When the composition according to the invention is used for intensifying the therapeutic activity of a pharmacologically active substance, said pharmacologically active substance can be introduced into the inner sphere of the coordination compound according to the invention as a ligand. It can also be located in the outer sphere (e.g., as a counterion). Said substance can also be contained in an aqueous solution of the composition according to the invention in the form of free molecules. In the aforementioned cases the composition according to the invention and the pharmacologically active substances whose efficacy it intensifies are present in one dosage form. This case means, of course, that they can be administered simultaneously.

As an alternative, the coordination compound of a d-metal or of several d-metals and the pharmacologically active substance whose efficacy is to be intensified with their aid can be in separate dosage forms. Administration in separate dosage forms can be effected simultaneously (simultaneous administration of two solid dosage forms, e.g. tablets, simultaneous injection, in particular in one syringe), or successively, when the patient is given or administered the first dosage form first, and then the second dosage form. The interval between administrations is preferably not more than 1 hour. The optimal order of administration depends on the pharmacokinetics and pharmacodynamics of the pharmacologically active substance whose efficacy is to be intensified (rate of absorption, distribution, rate of elimination, characteristics of cellular and organ tropism or systemic tropism) and can be selected for each concrete substance individually.

It should be pointed out that the ligand in the proposed coordination compound must be a physiologically (biologically, pharmacologically) and pharmaceutically acceptable compound.

"Physiologically (biologically, pharmacologically) acceptable" means a compound that does not cause toxic or other undesirable effects when administered to a patient. The terms physiologically acceptable, biologically acceptable and pharmacologically acceptable are considered to be synonyms in the context of this description. It is also understood that all substances used are also pharmaceutically acceptable, i.e. they can be used as ingredients in dosage forms.

In the context of the present invention, "patient" means a human being or other mammal, bird, amphibian or fish, to which the dosage form is administered by some route or other.

Ligands, d-metals and other pharmacologically active substances can be tissue-specific or organ-specific. The ligand and the d-metal may also possess their own pharmacological activity. Therefore in order to increase the efficacy of the combination with a pharmacologically active substance, the ligand and the d-metal can be selected so that their tropism and/or pharmacological activity correspond to the tropism and/or pharmacological activity of said pharmacologically active substance (see Fig. 11).

The amount of coordination compound of a d-metal to be introduced, classed among the biometals, is determined by the proportion by mass of the biometal in the composition of the coordination compound, which can correspond to or can be below the daily requirement of the biometal used, otherwise the amount of d-metal introduced in the composition of the coordination compound is determined by the need to achieve a therapeutic result. The amount of coordination compound of a d-metal introduced not classed among the biometals is determined by the proportion by mass of d-metal in the composition of the coordination compound and must be below their maximum permitted values, otherwise the amount of d-metal introduced in the composition of the coordination compound is determined by the need to achieve a therapeutic result.

"Low concentration" is a concentration below $10^{-3}$ mol/liter, and "ultra-low" concentration is a concentration below $10^{-6}$ mol/liter.

In general, all compositions and methods according to the invention can alternatively comprise, consist of or essentially consist of any suitable components and stages disclosed in this description, and such methods according to the invention can additionally or alternatively exclude any component, or stage, or object, that is used in an agent or a method known from the prior art, or that is not necessary for achievement of the technical result of the present invention.

The invention will now be explained with concrete examples.

Example No. 1. Production of a coordination compound of Fe(II) with cysteine

Production of an iron coordination compound, coordinated with the sulfur-containing ligand cysteine, is carried out in several successive stages. In the first stage it is necessary to carry out the process of formation, in solution, of aqua-hydroxo complexes of an inorganic d-metal compound suitable for coordination with a nitrogen-sulfur-containing organic molecule (in the present case, cysteine), for which 137.67 mg of $FeS0_4 \cdot 7H_2O$ or 194.19 mg of $(NH_4)_2Fe(SO_4)_2 \cdot 6H_2O$ was dissolved in 250 ml distilled water and held for 1-3 minutes.
In the second stage 1 ml of the solution obtained was added to a solution containing 1.2 g cysteine, dissolved in 100 ml water.
Next, vacuum-sublimation drying of the resultant reaction mixture was carried out, during which there is formation of a pale yellow substance, in which the ratio of coordination compound to organic molecule is 1/5000.
On completion of vacuum-sublimation drying, the substance is ready for use.

Example No. 2. Production of a coordination compound of Fe(III) with cysteine

Dissolve 44.62 mg of $FeCl_3 \cdot 6H_2O$ or 79.60 mg of $(NH_4)Fe(SO_4)_2 \cdot 12H_2O$ in 10-15 ml water, add 45-68 mg of $NaO_2CCH_3 \cdot 3H_2O$. After leaving the solution to stand, dilute it with water to 500 ml. 1 ml of the solution obtained was added to a solution containing 1 g of cysteine dissolved in 100 ml water, and the solution was transferred to the apparatus for vacuum-sublimation drying.
In the pale yellow substance obtained, the ratio of coordination compound to organic molecule is 1/5000.
On completion of vacuum-sublimation drying, the substance is ready for use.

Example No. 3. Production of a coordination compound of copper with cysteine

164.78 mg of copper(II) acetate $(Cu(O_2CCH_3)_2 \cdot H_2O)$, which in solution is in the form of a dimer complex with a "Chinese lantern" framework, was dissolved in 100 ml water and 1 ml of the solution obtained was transferred to a solution of one gram of cysteine in 50 ml water, and was left to stand for 1-3 min. Next, vacuum-sublimation drying of the reaction mixture was carried out, in the course of which a white substance formed in the reaction mixture.
In this case the ratio of coordination compound to organic molecule is 1/1000.
On completion of vacuum-sublimation drying, the substance is ready for use.

Example No. 4. Production of a coordination compound of copper with cysteine

Dissolve 351.77 mg of copper(II) chloride $(CuCl_2 \cdot 2H_2O)$ and 338.54 mg of anhydrous sodium acetate in 10-15 ml water, leave to stand for several minutes at room temperature and bring the volume of the resultant solution up to 250 ml. Add 1 ml of a solution of the coordination compound to a solution of cysteine (approx. 1 g in 100 ml water), leave to stand for 1-3 min. Then vacuum-sublimation drying of the reaction mixture is carried out, in the course of which a white substance forms in the reaction mixture.
The ratio of coordination compound to organic molecule in the substance is 1/1000.
On completion of vacuum-sublimation drying, the substance is ready for use.

Example No. 5. Production of a palladium coordination compound with cysteine

Dissolve 234.66 mg $(NH_4)_2[PdCl_4]$ in 50 ml water, leave to stand for 5-10 min and bring up to the 100 ml mark. Transfer 1 ml of the solution obtained to a solution containing 1 g cysteine in 100-150 ml water. Next, vacuum-sublimation drying of the reaction mixture is carried out, in the course of which a pale yellowish-green substance forms in the reaction mixture.
The ratio of coordination compound to organic molecule in the substance is 1/1000.
On completion of vacuum-sublimation drying, the substance is ready for use.

Example No. 6. Production of a coordination compound of copper with glycine

398.94 mg of copper(II) acetate monohydrate $(Cu(O_2CCH_3)_2 \cdot H_2O)$, which in solution is in the form of a dimer complex with a "Chinese lantern" framework, was dissolved in 100 ml of water and 1 ml of the solution obtained was transferred to a solution of 1.5 g glycine in 50 ml water, and was left to stand for 1-3 min. Next, vacuum-sublimation drying of the reaction mixture was carried out, in the course of which a white substance forms in the reaction mixture.
The ratio of coordination compound to organic molecule in the substance is 1/1000.
On completion of vacuum-sublimation drying, the substance is ready for use.

Thus, examples 1-6 describe the production of coordination compounds in excess of ligand, which makes it possible to avoid both loss of d-metal and of coordination compound when producing small and ultra-small amounts and subsequent use thereof, for example through nonspecific sorption on the walls of the vessels used, and measure out small and ultra-small amounts of the coordination compound by the widely used gravimetric and volumetric methods.

Examples of determination of the intrinsic biological activity of the coordination compounds using cultured cells and experimental animals are presented below.

The biological activity of the investigational coordination compounds was studied using "in vivo" models employing a specialized cell culture A431, macrophages, in animals. The cells of the human epidermoid carcinoma line A431 have an extraordinarily high level of expression of the epidermal growth factor receptor, making it possible to interpret the results obtained with a high confidence level from the activation status of the EGF receptor and EGF-dependent signalling systems and the biochemical processes regulated by them. Macrophages are one of the most characterized cells of the immune system, making it possible, during assessment of test compounds, to characterize the components of their biological activity with a high confidence level.

The advantage of using permanent cell lines is standardization of their culture conditions, which makes it possible to minimize unimportant or random effects, not connected with the test substance. Standardization of the cell culture conditions is achieved using standard characterized nutrient media, certified equipment that maintains optimal physiological conditions of cell growth, and standardization of the growth rate of the cells and their density before the experiment. However, a cellular model does not reflect all of the interactions of cells in the structure of a tissue, organ, organ system, the organism as a whole, which can have a significant influence on the biological activity of chemical compounds. In this connection, experiments on animals made it possible to assess the stability of the biological activity of coordination compounds when introduced into the organism.

<u>Example 7. Influence of coordination compounds on activation of the epidermal growth factor receptor, MAP kinase ERK1,2 and induction of hemoxygenase activity</u>

Purpose of the work. To investigate the influence of coordination compounds on cellular processes through action on cell-surface receptors. The test compounds used were cysteine (C), glycine (G), coordination compounds of cysteine with copper (C-Cu) and glycine with copper (G-Cu), C-Cu coordination compound with excess of cysteine (C-Cu:C) and G-Cu coordination compound with excess of glycine, ratio of C-Cu:C to G-Cu:G = 1:1000.

<u>Preliminary treatment of the preparations for carrying out the investigation</u>

The test compounds were stored at +4˚C; directly before the start of the experiment the substances were dissolved in deionized water (super Q). The prepared solution can be stored for not more than 5 hours at +4˚C.

<u>Route of introduction</u>

The compounds were added to the cell culture medium up to the final concentration to be investigated.

<u>Number of doses</u>

The preparation is added to the cells once for the stated period of time.

<u>Concentrations</u>

Cysteine 0.015 μmol/ml,
**[0106]** Cysteine coordination compound:cysteine (C-Cu:C) 1:1000 0.015 μmol/ml
Cysteine C-Cu coordination compound 0.015 nmol/ml,

Glycine 0.015 μmol/ml,

Glycine coordination compound:glyc.ine (G-Cu:G) 1:1000 0.015 μmol/ml
Glycine coordination compound G-Cu 0.015 nmol/ml.

<u>Time of action of the preparation</u>

0 min, 5 min, 10 min, 30 min, 1 h, 2 h, 4 h, 8 h.

Cell line used

Human epidermoid carcinoma cells of line A431, obtained from the All-Russian Cell Culture Collection (Cytology Institute of the Russian Academy of Sciences, St Petersburg).

Cell culture conditions

The cells are cultivated in a $CO_2$-incubator (New Brunswick Scientific) at +37°C and at $CO_2$ content of 5%. In these conditions the cells grow to a monolayer culture and are submitted to the action of the test compounds.

Cell culture medium

The cells are cultured using DMEM medium ("PanEco" Ltd., Moscow) with addition of gentamicin K (80 mg/l), L-glutamine (300 mg/l) and fetal serum (RAA, Austria) to a final concentration of 10%. Twenty-four hours before the start of the experiment the cells are transferred to a medium with lower serum content - 0.5%.

Dynamics of cell growth

The cells are sown on plastic Petri dishes (Nunc) at a concentration of 40000/$cm^2$ and are cultured to a subconfluent state (approx. 80-90% of the monolayer) for 2 days. Then the cells are transferred to a medium with reduced serum content for a day.

Method of cell lysis

After completion of stimulation, the cells are washed with phosphate-salt buffer (pH 7.4) on ice. To obtain the total lysate, the cells are scraped off in a buffer containing 20 mM Tris, pH 7.4, 150 mM NaCl, 1 mM $Na_3VO_4$, 1 mM EDTA, 1 mM EGTA, 0.5 mM PMSF, 1 μg/ml each of leupeptin, aprotinin and pepstatin, 0.5% Nonidet P-40, 1% Triton X-100, and are incubated for 10 min at 4°C. The material obtained is passed through a 26G needle 5 times and centrifuged for 5 min at 10000 g. 1/4 of the volume of buffer is added to the supernatant for electrophoretic samples (40 mM Tris, pH 6.8, 10% SDS, 20% β-mercaptoethanol, 40% glycerol, bromophenol blue 0.05%), stirred and heated for 5 min at 100°C.

Determination of protein concentration

Determination of the concentration of protein in the samples prior to application for electrophoresis is carried out using the reagents from the RC-DC Protein Assay kit (BioRad) on a BioMate 3 spectrophotometer (Thermo Electron Corporation).

Electrophoresis and electromigration

Electrophoretic separation of proteins is carried out in 7.5% polyacrylamide gel for analysis of epidermal growth factor receptor and in 12% gel for analysis of MAP kinases ERK1,2 according to the standard protocol. Samples containing an equal amount of protein are applied to the gel. Subsequent electromigration of the separated proteins onto a nitrocellulose membrane (Hybond ECL, Amersham) is carried out in a buffer containing Tris 0.58%, glycine 0.29%, dodecylsulfate Na 0.035% and methanol 20%. Ponceau S (Sigma) is used for visualization of the protein bands.

Immunoblotting

Immunoblotting is carried out in accordance with the protocol of ECL Western blotting protocols (Amersham). All procedures are carried out at room temperature. The nitrocellulose membrane is washed in a solution containing 10 mM Tris, pH 7.6, 100 mM NaCl and 0.1% Tween 20 (TBS-T) for 10 min. Then the membrane is incubated in 1% solution of BSA (Sigma) or 5% solution of skim milk (Valio) on TBS-T buffer for 1 h, and then in a solution of primary antibodies in TBS-T buffer for 1-1.5 h. Then the membrane is washed in TBS-T 5 times for 5 min each time, put in a solution of secondary antibodies, in TBS-T buffer, containing 5% skim milk, and incubated for 1 h. After washing the membrane (5 times, 5 min each time, TBS-T), the proteins that have bound to the antibodies, are revealed by enhanced chemiluminescence (ECL). The nitrocellulose membrane is incubated in ECL solution (Amersham) on the basis of 0.125 ml/$cm^2$ for 1 min and the chemiluminescence is recorded by exposure of a Kodak X-OMAT AR X-ray film.

Antibodies

Specific detection of proteins on the membrane uses primary monoclonal antibodies to phosphotyrosine (PY20, Transduction Laboratories, USA), and polyclonal rabbit antibodies to tyrosine-phosphorylated ERK1,2 (Cell Signaling Technology, USA). For additional identification of the active forms of the investigated proteins detected during the work, polyclonal rabbit antibodies to EGF receptor, and polyclonal rabbit antibodies to ERK1,2 (Cell Signaling Technology, USA) are used. The secondary antibodies used in immunoblotting are goat antibodies, obtained against mouse immunoglobulins, conjugated with peroxidase (GAM-HRP) (Sigma, USA) and goat antibodies developed against rabbit immunoglobulins, conjugated with horseradish peroxidase (GAR-HRP, Cell Signaling Technology, USA). Primary polyclonal rabbit antibodies to hemoxygenase 1 (Santa Cruz, USA) are used for specific detection of hemoxygenase. Antibodies obtained against rabbit immunoglobulins, conjugated with peroxidase (GAR-HRP) (Santa Cruz, USA), are used as secondary antibodies in immunoblotting.

Method of removal of antibodies from the membrane (stripping)

To remove antibodies that have bound to the nitrocellulose membrane and stain it again with other antibodies, the membrane is incubated in a solution containing 60 mM Tris-HCl (pH 6.8), 2% SDS and 100 mM 2-mercaptoethanol, for 30 min at 50°C with shaking.

Methods of densitometry and statistical analysis of the results

The X-ray films with the results were scanned using Photoshop 6.0 software, densitometry of the bands was carried out using the Scion Image 4.0.2. software, the values obtained were divided by the control value, and were averaged for three similar experiments (repeats). Graphs were constructed in Microsoft Excel 2000 9.0, plotting time on the abscissa, and the ratio of the experimental values to the control value on the ordinate.

Results for the influence of the test compounds on the epidermal growth factor receptor

According to the results obtained, cysteine causes activation of the EGF receptor with long periods of action - from 1 hour to 4-8 hours - by a factor of 8-10 (Fig. 3(1)).

The C-Cu coordination compound has a more pronounced influence on the tyrosine kinase activity of the epidermal growth factor receptor (Fig. 3(2)).

In contrast to the receptor's reaction to the action of cysteine, its effect in the composition of the coordination compound is manifested by activation of the receptor within the first hour after introduction of the coordination compound. Similar regularity of the receptor's response appears on introduction of the coordination compound in excess of cysteine (Fig. 3(3)).

The combined effect of cysteine and its coordination compound, used at 1000:1, alters the manifestation of tyrosine kinase activity of the epidermal growth factor receptor. In particular, its intensification should be noted, 5 and 60 minutes after introducing the test substance. The tyrosine kinase activity of the EGFR after 10 and 30 min, 2 and 4 hours did not differ significantly from that under the action of the coordination compound. After 8 hours, the tyrosine kinase activity of the EGFR was similar to that under the action of cysteine. Bearing in mind that cysteine is not a neutral molecule with respect to many biological targets, acting directly or indirectly, based on the results of these experiments it can only be said extremely relatively that the coordination compound has a particular activity with respect to the epidermal growth factor receptor. Moreover, with combined use of cysteine and its coordination compound we may only say that there are certain features in the change in the response of the epidermal growth factor receptor to this action. However, use of the coordination compound of glycine and copper (G-Cu), independently and in combination with glycine, in a series of experiments of a similar nature, provided certain results, according to which the coordination compound possesses independent biological activity (Fig. 4).

In these experiments there is intensification of the effect of the coordination compound when it is combined with excess glycine (Fig. 4, diagram 3). This intensification of activity may result from having a larger amount of coordination compound in the incubating medium. When working with low concentrations there is the danger that some of the substance may be lost as a result of sorption on the walls of the vessels and pipettes. Such losses are slight, but when using substances in ultra-small amounts they may lead to almost complete binding of the substance. In this case excess of the organic molecule helps to ensure that the incubating medium has the planned concentration of the test substance.

It can be stated, on the basis of the results obtained, that a coordination compound of a d-metal and a nitrogen-containing or sulfur-containing organic molecule possesses intrinsic biological action, which may or may not coincide with the action of the organic molecule on a particular biological target.

The receptor is the primary target for the action of many factors in the environment. Activity of the receptor still

does not mean formation of a cellular response. In transmission of the signal generated by the receptor, it is important to include mediators, which among other things predetermine the character of the cellular response. The MAP kinases ERK1,2 are among the key mediators in transmission of the regulatory signal from the epidermal growth factor receptor.

The nature of the change in activity of MAP kinases ERK1,2 in response to activation of the epidermal growth factor receptor is shown in Fig. 5.

It is clear that both cysteine and its coordination compound alter the activity of MAP kinases ERK1,2, which does not contradict other data on the influence of cysteine on various targets. It is notable that the action of the cysteine coordination compound led to greater activation of these enzymes, and combined use of the coordination compound in combination with excess cysteine led in fact to summation of their effects at the times when reaction of MAP kinases ERK1,2 was observed (Fig. 5 diagram 3).

If the organic molecule does not have a pronounced action on the receptor, and hence on activation of the signal-transmitting proteins, the action of the coordination compound, used separately or in combination with excess of bindable organic molecule, becomes evident. Thus, in a series of experiments, with glycine as the organic molecule, the intrinsic influence of which was barely detected on the epidermal growth factor receptor (Fig. 4), activation of MAP kinases ERK1,2 was also not determined (Fig. 6, diagram 1).

However, in the case of an effect of the glycine coordination compound - G-Cu, as well as its coordination compound, introduced in excess of the organic molecule (Fig. 6, diagrams 2 and 3 respectively), the relationship typical of the action of the cysteine coordination compound, applied separately or in an excess of the organic molecule, was observed. When there is action on the signal-transmitting proteins, the character of the cellular response, predetermining the cellular reaction to the action, is important. The cellular response dependent on MAP kinases ERK1,2 is, among other things, linked to the expression of hemoxygenase 1, a protein belonging to the enzymes of the second phase of detoxication of xenobiotics. The results of investigation of the expression of hemoxygenase 1 are shown in Fig. 7.

It is clear that expression of hemoxygenase 1 occurs in the case of the action of the C-Cu coordination compound and cysteine in combination with the coordination compound in the ratio 1000:1 (Fig. 7) and there are certain regularities in its manifestation. Thus, expression of the enzyme is of a wavelike character, being manifested 9-11 hours after manifestation of the activity of the receptor and of the signal-transmitting proteins. Expression of hemoxygenase-1 was not detected when a selective inhibitor of the epidermal growth factor receptor was used. Thus, the results obtained point to independent activity of the coordination compounds, which may be combined with the action of the organic molecule, or may be separate. In the spectrum of the investigational coordination compounds, their biological activity was detected in induction of the enzyme of the second phase in the detoxication of xenobiotics, hemoxygenase-1, the activity of which is linked to a set of biochemical reactions in the reuse of iron in the cell and its subsequent use in reactions of synthesis of heme, a molecule that is an indispensable component of the heme-containing proteins of the respiratory chain, myoglobin, hemoglobin and key enzymes of various types of metabolism.

In this connection, the possibility of participation of coordination compounds in the processes of induction of other enzymes in the second phase of detoxication of xenobiotics and capacity for combining with agents that stimulate hemopoiesis was assessed.

<u>Example 8. Influence of a coordination compound on the activity of enzymes in the second phase of detoxication of xenobiotics in the liver cells of an animal</u>

Purpose of the work. To investigate the influence of coordination compounds on the activity of enzymes in the second phase of detoxication of xenobiotics in the liver cells of an animal.

The test compounds used were cysteine (C), and a cysteine coordination compound with copper (C-Cu) with excess of cysteine in the ratio C:C-Cu = 1000:1.

The investigation was carried out on random-bred male white rats weighing 140-160 g, from the "Rappolovo" breeding station of the Russian Academy of Medical Sciences, hepatotoxic action in which was caused by daily, for 10 days, application of cyclophosphane (CP) at a dose of 20 mg/kg subcutaneously in physiological solution.

The experimental animals were divided into 4 groups.

No. 1 - intact animals, which received injections of the solvent of the test compounds (physiological solution) (solvent control);

No. 2 - animals that received CP, and were then administered physiological solution as the therapeutic agent (control);

Test groups:

No. 3 - animals that received the test compound cysteine (C) in physiological solution intraperitoneally at a dose of

10 mg/kg for 10 days, 30 min after introduction of the toxicant CP.

No. 4 - animals that received cysteine (C) in combination with the coordination compound of cysteine and copper (C-Cu:C, ratio of cysteine to coordination compound 1000:1) in physiological solution intraperitoneally at a dose of 10 mg/kg (amount of coordination compound $8.3 \times 10^{-8}$M/kg) for 10 days, 30 min after introduction of the toxicant CP. Enzymes in the second phase of detoxication of xenobiotics in the cytosolic fraction of liver cells were investigated: glutathione-S-transferase (EC 2.5.1.18), glutathione peroxidase (EC 1.11.1.9), glutathione reductase (EC 1.6.4.2), glucose-6-phosphate dehydrogenase (EC 1.1.1.49.)

Results of the investigation

The results of investigation of a set of molecular reactions that provide tolerance to the action of toxic substances provide evidence of the ability of cysteine (C), and of its coordination compound (C-Cu) to induce the activity of enzymes in the second phase of detoxication of xenobiotics - glutathione reductase (EC 1.6.4.2), glutathione peroxidase (EC 1.11.1.9), glutathione-S-transferase (EC 2.5.1.18), as well as the associated metabolism of the reduced glutathione (see Table 1).

Table 1 Influence of the coordination compound of cysteine and copper on the ability of cysteine (ratio of cysteine to coordination compound 1000:1) to induce the activity of enzymes in the second phase of detoxication of xenobiotics in liver cells of mongrel white rats on repeated application of cyclophosphane at a dose of 20 mg/kg for 10 days (Activity of the enzymes in μmol/(min · g protein, reduced glutathione - μmol/g protein)

| Study group | Values of the index analyzed | | | | |
|---|---|---|---|---|---|
| | GR | GP | GST | G-6-PDG | GSH |
| Control | 271.3±15.8 | 15.2±0.4 | 2281±187 | 181.6±12.9 | 13.68±0.62 |
| Uncorrected | 175.3±11.7* | 13.8±0.2* | 1727±86* | 86.2±7.7* | 9.61±0.02* |
| cysteine-copper:cysteine 1000:1 | 270.8±12.6 # | 27.9±2.2*# | 2857±120*# | 158.1±13.3 # | 11.21±0.56* |

* - significance of difference p<0.05 compared with control group
\# - significance of difference p<0.05 compared with group of poisoned animals without correction
GR - glutathione reductase (EC 1.6.4.2)
GP - glutathione peroxidase (EC 1.11.1.9)
GST - glutathione-S-transferase (EC 2.5.1.18)
G6PDG - glucose-6-phosphate dehydrogenase (EC 1.1.1.49)
GSH - reduced glutathione

Nano-amounts of the coordination compound enhanced the ability of cysteine to induce activity of the enzymes in the second phase of detoxication of xenobiotics, and increased the rate of metabolism of the key metabolite associated with them: reduced glutathione.

Thus, nano-amounts of the coordination compound, introduced in combination with a bindable organic molecule, used in excess and possessing the capacity to induce the activity of enzymes in the second phase of detoxication of xenobiotics, enhanced its toxicity-modifying and, accordingly, cytoprotective action by induction of enzymes in the second phase of detoxication of xenobiotics.

Example 9. Influence of a palladium coordination compound on the activity of calcium channel inhibitors

Purpose of the work. To investigate the influence of coordination compounds on the activity of ion channels in the cell membrane.

The following compounds were investigated: the selective calcium channel inhibitor nifedipine, palladium coordination compound with cysteine (Pd-C), which was introduced in excess of cysteine in the ratio of cysteine to palladium coordination compound (C:Pd-C) of 1000:1.

Preliminary treatment of the preparations for carrying out the investigation

The test compounds were stored at +4°C; immediately before the start of the experiment the substances were

dissolved in deionized water (super Q). The prepared solution can be stored for not more than 5 hours at +4°C. The compounds were added to the cell culture medium up to the final concentration to be investigated.

The preparation is added to the cells once for the stated period of time.

Cell line used, culture conditions

The experiments were carried out on cultured resident peritoneal macrophages from the rat. Resident macrophages were isolated from the peritoneal cavity of rats weighing 200-300 g by the method described previously [5, 9]. Immediately after isolation the cells were of spherical shape with a diameter of 10-20 μm. A suspension of the cells was put in culture dishes, containing 10x10 mm quartz coverslips. The cells on the coverslips were cultured in medium 199 (pH 7.2) with addition of 20% bovine blood serum, solution of glutamine (3%), penicillin (100 U/ml) and streptomycin (100 mg/ml) for 1-3 days at 37°C. After staining with α-naphthyl acetate esterase [7], it was established that at least 96% of the cells in the monolayers were macrophages. The experiments were carried out at room temperature 20-22°C for 2-3 days of cell culture.

The quartz coverslips with the cells were put in a test chamber filled with physiological solution with the following ionic composition (mM): NaCl - 140, KCl - 5, $CaCl_2$ - 1, $MgCl_2$ - 1, HEPES-NaOH - 5; pH 7.3-7.4 (Alonso-Torre, Trautmann, 1993). The calcium-free medium contained 0 mM $CaCl_2$ and 1 mM EGTA.

Reagents from the company Sigma were used for the tests. Stock solutions of thapsigargin (500 μM) and nifedipine (20 mM) were prepared in dimethylsulfoxide. Stock solutions of cysteine (0.45 μmol/ml), cysteine in combination with the cysteine coordination compound and palladium of ratio 1000:1 (0.45 μmol/ml), ATP (100 mM), were prepared with water.

The influence on $[Ca^{2+}]_i$ was investigated: inhibitory activity of nifedipine cysteine (0.015 μmol/ml) palladium coordination compound with cysteine (Pd-C), which was introduced in cysteine excess in the ratio 1:1000 (Pd-C:C) to a final concentration of 0.015 μmol/ml.

Test procedure

The intracellular calcium concentration ($[Ca^{2+}]_i$) was measured using a Fura-2AM fluorescent probe. The macrophages were incubated for 45 min in physiological solution containing 2 μM Fura-2AM at room temperature (to prevent endocytosis of the micelles of Fura-2AM, which takes place at 37°C) [3]. The coverslips with the stained cells were washed with physiological solution and transferred to the test chamber, which was on the stage of a "Lumam-KF" luminescence microscope. Fluorescence of Fura-2 was excited at 337 nm with a type LGI-503 nitrogen laser. The laser was positioned to the side of the microscope at an angle of 30° to the test chamber, making it possible to direct the laser beam directly onto the object. Fluorescence intensity was recorded by means of the SFN-10 spectrophoto-adapter at a wavelength of 510 nm. The signal from the FEU-79 was amplified with a specially designed amplifier and was recorded on an IBM PC computer using original software. A 10 X 0.40 objective was used in the experiments. At this magnification, there were 40-50 cells in the area for photometric measurement. To avoid photo-burnoff, measurements were performed every 20 s with irradiation of the object for 2.5 s. On addition of ATP and UTP the cells were irradiated continuously until maximum fluorescence was reached. The values of $[Ca^{2+}]_i$ were calculated from the Grynkiewicz equation [6]:

$$[Ca^{2+}]_i = K_d(F-F_{min})/(F_{max}-F),$$

where F is the observed fluorescence intensity; $F_{max}$ is the fluorescence of dye saturated with $Ca^{2+}$; $F_{min}$ is the fluorescence of dye free from $Ca^{2+}$ (in the calcium-free medium).

At 20°C and pH 7.1-7.2, the dissociation constant, $K_d$, for the Fura-2AM:$Ca^{2+}$ complex is 135 nM. $F_{max}$ was measured after adding 10 μM ionomycin or 25 μM digitonin to the cells, which were in a medium containing $Ca^{2+}$. Treatment of the cells with digitonin allows the $Ca^{2+}$ ions to pass freely through the plasma membrane, without affecting the permeability of the membranes of the mitochondria and endoplasmic reticulum. After the signal had stabilized, 5 mM EGTA was added and the fluorescence of the dye was determined in nominally calcium-free medium ($F_{min}$). The level of intrinsic fluorescence was subtracted after adding $MnCl_2$ solution (100 μM) to the macrophages. $Mn^{2+}$ displaces $Ca^{2+}$ from the complex with Fura-2, and the fluorescence of the complex of the dye with $Mn^{2+}$ is 100 times lower than the fluorescence of the complex of Fura-2 with $Ca^{2+}$. For Fura-2, $F_{min} = F_{max} / 3$.

The research was based on two experimental approaches. The first investigated the effect of the pharmacological agents on the $Ca^{2+}$ response caused by ATP, UTP, thapsigargin or cyclopiazonic acid (CPA) in macrophages that are in normal physiological solution. The agents were introduced either before the action of the agonists, or afterwards, during the plateau phase of the $Ca^{2+}$ signal, reflecting entry of $Ca^{2+}$ from the environment. In the second variant of tests

for detecting and intensifying entry of $Ca^{2+}$ into the cells, the following experimental scheme was used ($Ca^{2+}$-free/$Ca^{2+}$-reintroduction protocol). The macrophages were incubated in nominally calcium-free medium, then they were acted upon by one of the agonists, causing mobilization of $Ca^{2+}$ from the intracellular stores. After adding 2 mM $Ca^{2+}$ to the external medium and restoring the physiological gradient of $Ca^{2+}$ concentration, a rapid increase in $[Ca^{2+}]_i$ is observed, reflecting entry of $Ca^{2+}$ into the cell. Next we investigated the influence of pharmacological agents added before introduction of the agonists, before introduction of $Ca^{2+}$ or during developing entry of $Ca^{2+}$ from the environment.

Results for the influence of the test compounds on the intracellular Ca2+ concentration and Ca2+ signals induced by ATP and thapsigargin, in rat macrophages

Addition of 200 μM ATP to the medium for incubation of peritoneal macrophages from the rat produces a two-phase $Ca^{2+}$ signal, consisting of a brief initial peak, connected mainly with mobilization of $Ca^{2+}$ from the stores, caused by activation of the $P_{2U}$ receptors, and a pronounced, long "plateau" phase. This plateau phase is caused by entry of $Ca^{2+}$ from the environment and apparently reflects simultaneous activation of $P_{2U}$ and $P_{2Z}$ receptors. Fig. 8 (1) shows a typical $Ca^{2+}$ signal, induced by extracellular ATP (200 μM) in a population of 40-50 macrophages, contained in normal physiological solution.

In response to the introduction of extracellular ATP, $[Ca^{2+}]_i$ increases from the baseline 75 ± 18 nM to a peak of 820 ± 105 nM. Then there is a slowly diminishing plateau phase, during which $[Ca^{2+}]_i$ is on average 460 ± 115 nM, 4 min after addition of ATP.

The specific inhibitor of endoplasmic $Ca^{2+}$-ATPases thapsigargin (0.5 μM) also gives rise to a two-phase $Ca^{2+}$ signal: a fairly quick peak, connected with mobilization of $Ca^{2+}$ from the stores, and a protracted phase, reflecting the depot-dependent entry of $Ca^{2+}$ from the environment. Fig. 8 (2) shows a typical $Ca^{2+}$ signal induced by thapsigargin in macrophages, contained in normal physiological solution.

To detect and amplify the phase of entry of $Ca^{2+}$ into the cell, experiments were carried out using calcium-free medium. After stimulation of the macrophages with 200 μM ATP (Fig. 8(3)) or 0.5 μM thapsigargin (Fig. 8(4)) in nominally calcium-free medium (0 mM $CaCl_2$ and 1 mM EGTA), entry of $Ca^{2+}$ was induced by adding 2 mM $Ca^{2+}$ to the external medium.

Fig. 9 shows the influence of cysteine on $[Ca^{2+}]_i$ at rest and the $Ca^{2+}$ signals induced by 200 μM ATP (1, 2) and 0.5 μM thapsigargin (3) in macrophages, contained in normal physiological solution (1) or in nominally calcium-free medium (2, 3).

The data obtained provide evidence that cysteine is able to increase $[Ca^{2+}]_i$ to 180 ± 19 nM by mobilization of calcium from the intracellular stores. Emptying the intracellular calcium stores reduces the effect from the action of ATP. Thapsigargin completely cancels the effect of the ability of cysteine to mobilize calcium from the stores.

Fig. 10 shows the influence of a coordination compound (CC) of palladium with cysteine on the intracellular calcium concentration ($[Ca^{2+}]_i$) at rest and the $Ca^{2+}$ signals caused by ATP. The coordination compound cancels the inhibitory effect of the selective calcium channel inhibitor - nifedipine (1), and the effect of the coordination compound itself is suppressed by the reducing agent dithiothreitol (2).

The data obtained show that Pd-C:C is able to increase $(Ca^{2+}]_i$ to 240 ± 28 nM by mobilization of calcium from the intracellular stores. Emptying the intracellular calcium stores reduces the effect from the action of ATP. Pd-C:C stabilize the flow of calcium into the cell from the medium, which is expressed by suppression of the action of the calcium channel inhibitor nifedipine on this process. Dithiothreitol cancelled the stabilizing influence of Pd-C:C itself on the functioning of the calcium channels.

Thus, the coordination compound is able to intensify the action of some compounds on the cells, and in the present experiment this was cysteine, with respect to which the coordination compound acted as a synergist, and cancel the influence of other compounds (in this experiment - nifedipine), with respect to which the coordination compound acted as an antagonist.

The results in examples 7-9 show that coordination compounds display biological activity at concentrations characteristic of the action of bioregulators (cytokines, hormones) and other biologically active substances (alkaloids, vitamins etc.). Modulation of the activity of cell-surface receptors and ion channels indicates that it is possible to influence extracellular and intracellular receptors, transport proteins of the cytoplasmic and intracellular membranes, extracellular regulatory and transport molecules of a peptide nature, proteins of the cytoskeleton, autoimmune reactions; antigen binding and recognition, processes of exo- and endocytosis, chemotaxis, chemokinesis, cytokinesis; intercellular, matrix-cellular and humoral-cellular interactions. The results presented point to the capacity of coordination compounds to exert effects that can be utilized for creating pharmacological preparations with various kinds of pharmacological activity: cytoprotective, direct and indirect antioxidant, hemostimulating, antihypoxanthine, immunomodulating, toxicity-modifying, antiinflammatory and many others.

The following are examples of determination of the capacity of coordination compounds to influence the efficacy of various pharmaceutical preparations, including interferon, antiviral, antibacterial and hemostimulating agents on cultured cells and experimental animal models.

Example 10. Influence of an iron coordination compound on the hemostimulating activity of the lithium ion

Purpose of the work. To investigate the influence of coordination compounds on the hemostimulating effect of the lithium ion.

The test compounds used were the coordination compound Fe-C-Li, which was introduced in excess of the lithium salt of cysteine in the ratio 1:1000 (Fe-C-L:C-Li), the lithium salt of cysteine (C-Li), and lithium carbonate.

Preparation of solutions of the test compounds for carrying out the investigation

The white crystalline substances were stored at +4˚C; directly before the start of the experiment the substance was dissolved in physiological solution. The solution was sterilized by passage through 0.22 μm Millex-GS filters (Millipore) in a sterile laminar-flow cabinet.

Experimental model

The investigation was carried out on random-bred male white rats weighing 140-160 g, which were given a single dose of cyclophosphane at 100 mg/kg subcutaneously in physiological solution.

The experimental animals were divided into 4 groups.

No. 1 - intact animals, which received injections of the solvent of the test compounds (physiological solution) (solvent control);

No. 2 - animals that received an injection of CP, and were subsequently administered physiological solution as the therapeutic agent (control); Test groups:

No. 3 - animals that received an injection of cyclophosphane, and were subsequently administered Fe-C-Li:C-Li in physiological solution at a dose of 10 mg/kg as the therapeutic agent (amount of coordination compound $7.8x10^{-8}$M/kg);

No. 4 - animals that received an injection of cyclophosphane, and were subsequently administered C-Li in physiological solution at a dose of 10 mg/kg as the therapeutic agent;

No. 5 - animals that received an injection of cyclophosphane, and were subsequently administered lithium carbonate in physiological solution at a dose of 3 mg/kg as the therapeutic agent (the amount of lithium carbonate was calculated from the proportion by weight of lithium ion (.55) in 10 mg of lithium salt of cysteine; a similar amount of lithium is contained in 3 mg of lithium carbonate);

No. 6 - animals that received an injection of cyclophosphane, and were subsequently administered cysteine in physiological solution at a dose of 9.5 mg/kg as the therapeutic agent (the amount of cysteine was calculated from the proportion by weight of cysteine (≈9.5) in 10 mg of cysteine, which is equivalent to 9.5 mg of the hydrogenated form of cysteine);

The test preparations were administered on the third day after application of cyclophosphane.

Material for investigation

Blood for hematological investigation was collected from the caudal vein 3, 7 and 14 days after application of CP. At the end of each series (3, 7 and 14 days) the experimental animals were euthanized with an overdose of ether and blood was obtained from the caudal vein and bone marrow from the femur.

Indices analyzed

In this investigation we assessed the cellularity of the blood (number of erythrocytes, thrombocytes, leukocytes, lymphocytes and neutrophils, as well as ESR) and of the bone marrow with 7-day administration of the test compounds to hemodepressed animals.

Test results

The results of these investigations of cellularity of the blood are presented in Tables 1-3.

Cyclophosphane at a dose of 100 mg/kg causes fairly pronounced cytopenia with respect to all the formed elements of the blood with absolute and relative lymphopenia, which was most pronounced on the 14th day (1-3). The

coordination compound Fe-C-Li:C-Li has a pronounced hemostimulating effect, which was manifested in almost complete restoration of the cellularity of the blood. The action of cysteine, and of the lithium salt of cysteine, of lithium carbonate, in contrast to the iron coordination compound with the lithium salt of cysteine, which was administered in an excess of the lithium salt of cysteine, is manifested in the form of positive trends, which as a whole can be assessed as a hemostimulating effect (Tables 2-4).

Table 2. General blood analysis of male rats with cytopenia 3 days after application of cyclophosphane (100 mg/kg), untreated (physiological solution was administered), treated with hemostimulator ($Li_2HCO_3$), lithium salt of cysteine (C-Li) and lithium salt of cysteine in combination with the coordination compound of cysteine with iron (C-Li:Fe-C-Li) used in the ratio 1000:1

| Indices investigated | Intact (physiol. solution) | Cyclo-phosphane (physiol. solution) | Cyclo-phosphane (cysteine) | Cyclo-phosphane ($Li_2HCO_3$) | Cyclo-phosphane + C-Li | Cyclo-phosphane +Fe-C-Li:C-Li |
|---|---|---|---|---|---|---|
| Hemoglobin, (g/l) | 15.2±0.16 | 13.0±0.2 | 13.2±0.21 | 13.1±0.19 | 12.9±0.22 | 12.5±0.25 |
| Erythrocytes, $10^{12}$/l | 6.7±0.16 | 3.5±0.19 | 3.0±0.29 | 3.7±0.21 | 4.0±0.23 | 4.8±0.15 |
| ESR, mm/h | 2.5±0.85 | 2±0.37 | 2.2±0.30 | 2.4±0.42 | 3.3±0.80 | 3.2±0.54 |
| Thrombocytes , $10^9$/l | 374±28.5 | 231±35.3 | 220±45.3 | 256±45.3 | 387±17.4 | 502±36.4** |
| Reticulocytes % | 1.8±0.2 | 0.08±0.005* | 0.09±0.006* | 0.118±0.01* | 0.1±0.1** | 0.5±0.1** |
| Leukocytes, $10^9$/l | 9.2±0.35 | 3.58±0.26* | 3.26±0.31* | 3.75±0.46* | 4.38±0.31* | 4.49±0.29* |
| Segmented neutrophils, % | 16±2.5 | 18.7±2.4* | 17.6±2.1 * | 19.7±3.4* | 42±1.6** | 38±1.6 ** |
| Lymphocytes % | 75.5±1.4 | 71±1.8* | 68±2.8 * | 62±3.8 * | 45±1.6* | 51.3±0. ** |

* - p 0.05 - compared with biological control;
** - p 0.05 - compared with cyclophosphane control

Table 3.

General blood analysis of male rats with cytopenia 7 days after application of cyclophosphane (100 mg/kg) untreated (physiol. solution administered), treated with hemostimulator ($Li_2HCO_3$), lithium salt of cysteine (C-Li) and lithium salt of cysteine in combination with the coordination compound of cysteine with iron (C-Li:Fe-C-Li) used in the ratio 1000:1

| Indices investigated | Intact (physiol. solution) | Cyclo-phosphane | Cyclo-phosphane (cysteine) | Cyclo-phosphane ($Li_2HCO_3$) | Cyclo-phosphane + C-Li | Cyclo-phosphane -C-Li:C-Li |
|---|---|---|---|---|---|---|
| Hemoglobin (g/l) | 15.2±0.16 | 13.8±0.56 | 13.4±0.76 | 12.8±0.44 | 13.2±0.68 | 13.9±0.31 |
| Erythrocytes $20^{12}$/l | 6.7±0.16 | 3.5±0.25 | 3.1±0.33 | 3.6±0.29 | 3.2±0.18 | 6.1±0.08** |
| ESR, mm/h | 2.5±0.85 | 1.8±0.31 | 1.9±0.39 | 2.0±0.36 | 3.2±0.80 | 2.0±0.37 |
| Thrombocytes , $10^3$/l | 374±28.5 | 126±10.0** | 124±11.0** | 119±12.0** | 164±10.0** | 228±13.8** |
| Reticulocytes % | 1.8±0.2 | 1.0±0.1 | 0.9.0±0.2 | 1.1±0.1 | 1.2±0.2 | 1.63±0.2** |
| Leukocytes, $10^9$/l | 9.2±0.35 | 3.41±0.09* | 3.47±0.08* | 3.22±0.11* | 4.8±0.18** | 6.88±0.18** |

(continued)

| Indices investigated | Intact (physiol. solution) | Cyclo-phosphane | Cyclo-phosphane (cysteine) | Cyclo-phosphane ($Li_2HCO_3$) | Cyclo-phosphane + C-Li | Cyclo-phosphane -C-Li:C-Li |
|---|---|---|---|---|---|---|
| Segmented neutrophils, % | 16±2.5 | 32±1.9* | 30±1.8* | 33±1.7* | 50.4±0.6 ** | 63.3±1.6** |
| Lymphocytes % | 75.5±1.4 | 57.7±1.1* | 51.5±1.3* | 50.7±1.6* | 22±0.9 * | 61±0.9) * |

* - p 0.05 - compared with biological control;
** - p 0.05 - compared with cyclophosphane control

Table 4.

General blood analysis of male rats with cytopenia 14 days after application of cyclophosphane (100 mg/kg) untreated (physiol. solution administered), treated with hemostimulator ($Li_2HCO_3$), lithium salt of cysteine (C-Li) and lithium salt of cysteine in combination with the coordination compound of cysteine with iron (C-Li:Fe-C-Li) used in the ratio 1000:1

| Indices investigated | Intact (physiol. solution) | Cyclo-phosphane | Cyclo-phosphane (cysteine) | Cyclo-phosphane ($Li_2HCO_3$) | Cyclo-phosphane + C-Li | Cyclo-phosphane +Fe-C-Li:C-Li |
|---|---|---|---|---|---|---|
| Hemoglobin, (g/l) | 15.2±0.16 | 12.3±0.68 | 12.5±0.5 | 13.1±0.57 | 13.6±0.63 | 13.7±0.64 |
| Erythrocytes, $10^{12}$/l | 6.7±0.16 | 4.4±0.17 | 4.7±0.21 | 4.9±0.27 | 5.2±0.26 | 6.0±0.12 |
| ESR, mm/h | 2.5±0.85 | 2.3±0.61 | 2.1±0.73 | 2.4±0.65 | 2.7±0.49 | 2.8±0.70 |
| Thrombocytes, $10^3$/l | 368±21.5 | 393±56.9 | 387±54.9 | 401±59.9 | 390±38.3 | 598±72.9** |
| Reticulocytes, % | 1.8±0.2 | 1.2±0.2 | 1.1±0.4 | 1.4±0.3 | 1.3±0.3 | 2.0±0.2** |
| Leukocytes, $10^9$/l | 9.2±0.35 | 4.6±0.19** | 5.7±0.22** | 5.9±0.26** | 5.6±0.22** | 10.7±0.19** |
| Segmented neutrophils, % | 16±2.5** | 14.3±1.6** | 13.3±1.9** | 14.9±2.6** | 20.3±2.4 ** | 26±2.2** |
| Lymphocytes % | 75.5±1.4 | 73.4±1.8 | 72.1±1.6 | 69.3±0.9 | 72.5±1.4 | 73.4±1.1 |

* - p 0.05 - compared with biological control;
** - p 0.05 - compared with cyclophosphane control

Thus, a d-metal possessing characteristic biological activity and introduced in the composition of a coordination compound in nano-concentrations promoted manifestation of a hemostimulating effect of an agent, used at a dose that does not possess any significant biological action. In other words, combined use of ultra-low concentrations of the coordination compound and low concentrations of the hemostimulating agent and their combined action that was displayed as a therapeutic effect complies quantitatively with the criterion of low-dose drug therapy.

<u>Example 11. Influence of an iron Coordination compounds on the efficacy of interferon</u>

Purpose of the work. To investigate the influence of a coordination compound on the biological activity of interferon.

The test compounds used were an Fe-C coordination compound, which was introduced in an excess of cysteine in the ratio 1:1000 (Fe-C-:C), and interferon.

The method of assessment of the antiviral activity of interferon is based on determination of the minimum amount thereof that protects cells of line L-68 from the cytopathic action of the virus. The coordination compound was introduced into the cell incubating medium to a concentration of 0.0015 μmol/ml, 0.015 μmol/ml and 0.15 μmol/ml before introduction of interferon, together with interferon, or 10 min, 30 min and 60 min after introduction of interferon. The

interferon titer in the experiments was $4x10^{-4}$ U/ml, $8x10^{-4}$ U/ml, $1.6x10^{-5}$ U/ml, $3.2x10^{-5}$ U/ml, $6.4x10^{-5}$ U/ml, $1.28x10^{-6}$ U/ml, $2.56x10^{-6}$ U/ml, $5.12x10^{-6}$ U/ml. The antiviral activity of the preparations was assessed from the ability of live cells to absorb crystal violet. The amount of crystal violet absorbed was determined photometrically at a wavelength of 595 nm, after separating the fraction of live cells and extracting the dye with methanol. The amount of dye absorbed, proportional to the number of live cells, was expressed in units of optical density.

Preparation of the L-68 cell line for the experiment.

The L-68 cell line is a strain of diploid lung cells from a human embryo, obtained at the Moscow Research Institute of Virus Preparations of the Academy of Sciences of the Russian Federation from the lung cells of an 11-week human embryo, aborted from a woman aged 28 years, who had no oncologic or venereal diseases, hepatitis, tuberculosis, genetic or congenital abnormalities.

The seed bank of the L-68 strain of diploid cells is certified for the preparation of immunobiological preparations at the Moscow Research Institute of Virus Preparations of the Academy of Sciences of the Russian Federation together with the L.A.Tarasevich GISK (State Research Institute for Standardization and Control of Medical and Biological Preparations) of the Ministry of Health of the Russian Federation.

The cells of the L-68 line are cultured at 37˚C in complete growth medium I. Culture was carried out in 250-ml plastic flasks (of the Costar type). The cells are spread on the bottom of the flask, forming a monolayer with the morphology typical of diploid fibroblasts. The spread cells are transferred to a suspension with a special medium, consisting of equal parts of 0.02% Versene solution and 0.25% trypsin solution. For this, the complete growth medium is poured away from the flask with the formed monolayer of cells, the monolayer is washed twice with special medium (Versene solution with trypsin) and incubated at 37˚C for 5 min. During this time, the monolayer of fibroblasts detaches from the plastic. The detached cells are separated with complete growth medium, breaking up conglomerates of cells by repeated pipetting. The cells are transferred to a sterile centrifugation tube, and centrifuged at 1200 rev/min for 10 min. The supernatant that has formed is poured away and the cells are transferred to complete growth medium. Then the cells are counted in a Goryaev chamber and used in the tests.

For determination of activity and toxicity, cell cultures can be used that have undergone at least 20 and not more than 30 passages.

Preparation of vesicular stomatitis virus

This experiment uses lyophilized vesicular stomatitis virus (VSV), packaged under sterile conditions in sealed glass ampules.

The virus is grown on the L-929 cell line. For this, a previously titrated dose of the virus (infectious titer of VSV - maximum dilution of the virus, at which there is complete disruption of a monolayer of cells in 1 day at 37˚C) is added to a flask with a formed monolayer of cells in complete culture medium. The flask contents are cultured for 1 day at 37˚C, after which the culture medium is poured into a 50-ml sterile test tube and centrifuged at 2000 rev/min. Then the supernatant obtained, containing the vesicular stomatitis virus, is poured into ampules in 1 ml portions in sterile conditions and lyophilized.

Determination of the infectious titer of the virus

Prepared cells of the L-68 line, suspended in complete culture medium 1, are transferred to a 96-well plate (of the "Costar" type) at a concentration of $5 \cdot 10^4$ cells per well in a volume of 0.2 ml. After this, the plate is incubated for 1 day at a temperature of 37˚C in a $CO_2$-incubator in a 5% $CO_2$ atmosphere. During this time the cells spread in the wells, forming a continuous monolayer. After 24 hours, the culture medium is decanted in sterile conditions and previously prepared double dilutions of the virus in four parallels are transferred to the wells of the plate. The VSV virus is transferred in complete culture medium in a volume of 0.2 ml. Then the plate is incubated in the conditions described above. At the end of incubation (after 24 hours) the culture medium is decanted and 0.05 ml of 0.2% solution of crystal violet in 20% methanol is added to each well. After 10 min the dye is removed, the plate is washed under running water and dried. Then 0.1 ml of lysis buffer is added to the plate for elution of the dye into the solution. The intensity of staining is read on a microplate reader at a wavelength of 595 nm.

The infectious titer of the virus is the maximum dilution of the virus at which there is complete disruption of the monolayer of cells in the wells in 1 day in the stated conditions. The optical density of the solution in said wells will be minimal and close to the background value.

Determination of activity

Double dilutions (above and below the assumed titer) of a standard activity sample (42-28-119-96P, the L.A. Tarasevich GISK), the activity of which is expressed in international units IU, are prepared in complete growth medium. The dilutions of the standard are carried out in a 96-well plate ("Costar" type) in a volume of 0.1 ml. At least 4 wells are used per dilution. One row of the plate is left for control of the culture medium (4 wells) and for control of the dose of VSV virus (4 wells). Each of these wells receives 0.1 ml. After diluting the standard, prepared cells of the L-68 line, suspended in complete culture medium I at a concentration of $5\text{-}10^4$ cells per well in a volume of 0.1 ml, are transferred to the plate. Then, at defined intervals of time, the Fe-C coordination compound, at concentrations of 0.0015 μmol/ml, 0.015 μmol/ml and 0.15 μmol/ml, is transferred to some of the rows with the diluted standard. Then the plate is incubated for 1 day at a temperature of 37˚C in a $CO_2$-incubator in a 5% $CO_2$ atmosphere. During this time the cells spread in the wells, forming a continuous monolayer. After 24 hours the complete growth culture medium is decanted in sterile conditions and the VSV virus, the infectious titfer of which was determined previously, is transferred to the wells of the plate. The VSV virus is transferred in full growth medium in a volume of 0.2 ml. For control of the medium, 0.2 ml of medium of the same composition without the VSV virus is added to the wells. Then the plate is incubated in the conditions described above. At the end of incubation (after 24 hours) the culture medium is decanted and 0.05 ml of 0.2% solution of crystal violet in 20% methanol is added to each well. After 10 min the dye is removed, the plate is washed under running water and dried. In the wells used for control of the medium, the stained monolayer must not have any signs of disruption. Then 0.1 ml of lysis buffer is added to the plate for elution of the dye into the solution. The intensity of staining is read on a microplate reader at a wavelength of 595 nm.

The interferon titer is taken to be the reciprocal of the dilution of the preparation at which the cell culture in 50% of the wells was fully protected from the cytopathic action of the virus.

Test results

It was established experimentally that preliminary incubation of cells with the coordination compound did not affect the antiviral activity of interferon.

The results of experiments in which the coordination compound was introduced after interferon provide evidence of the ability of the coordination compound to increase the efficacy of interferon, provided the coordination compound was applied not earlier than thirty minutes after the interferon had acted upon the cells (see Table 5).

Table. 5. Influence of the coordination compound on the antiviral activity of interferon

| | | Control of medium | Fe-C + Standard of interferon α, administered after: | | | | | | | Standard of interferon α | | Virus control |
|---|---|---|---|---|---|---|---|---|---|---|---|---|
| | | | 10 minutes | | 30 minutes | | 60 minutes | | | | | |
| | Dilution of INFα | 1 | 2 | 3 | 4 | 5 | 6 | 7 | 8 | 9 | 10 | 11 | 12 |
| A | $4x10^{-4}$ | 2.666* | 2.691 | 2.625 | 2.633 | 2.618 | 2.634 | 2.631 | 2.622 | 2.596 | 2.674 | 2.633 | 0.147 |
| B | $8x10^{-4}$ | 2.703 | 2.688 | 2.606 | 2.643 | 2.652 | 2.658 | 2.666 | 2.664 | 2.657 | 2.656 | 2.649 | 0.213 |
| C | $1.6x10^{-5}$ | 2.703 | 2.681 | 2.666 | 2.614 | 2.625 | 2.608 | 2.599 | 2.629 | 2.559 | 2.673 | 2.546 | 0 |
| D | $3.2x10^{-5}$ | 2.707 | 2.394 | 2.356 | 2.37 | 2.465 | 2.249 | 2.261 | 2.204 | 2.355 | 2.164 | 2.333 | 0.073 |
| E | $6.4x10^{-5}$ | 2.721 | ***1.532*** | ***1.355*** | ***1.328*** | ***1.241*** | ***1.512*** | ***1.306*** | ***1.397*** | ***1.635*** | ***1.377*** | ***1.303*** | 0.23 |
| F | $1.28x10^{-6}$ | 2.731 | ***0.566*** | ***0.619*** | ***0.566*** | ***0.627*** | ***0.349*** | ***0.437*** | ***0.437*** | ***0.441*** | ***0.542*** | ***0.619*** | 0.227 |
| G | $2.56x10^{-6}$ | 2.728 | 0.247 | 0.391 | 0.475 | 0.379 | 0.258 | 0.167 | 0.252 | 0.421 | 0.358 | 0.302 | 0.176 |

* The efficacy is given in units of optical density. The higher the efficacy, the closer the digital value of the optical density of the test sample to the value of the optical density in the comparative sample and conversely, the lower the efficacy, the closer the value of optical density to the value of the control for activity of the virus.

It is clear that the coordination compound increased efficacy at interferon titers of $6.4x10^{-5}$ $1.28x10^{-6}$, which was reflected in a larger increase in optical density in the test in which the action of interferon took place together with the coordination compound (1) compared with the test in which interferon acted alone (2). This character of distribution of the value of the test index shows that in test 1 antiviral action of interferon was manifested with respect to a larger number of cells than in test 2.

To find a more reliable value of the size of the increase in the efficacy of interferon by the coordination compound, an experiment was carried out that yielded a larger volume of experimental data (see Table 6).

Table 6

| Quantitative values of the increase in efficacy of interferon (given in units of optical density) | | | |
|---|---|---|---|
| Dilutions of interferon α | Standard of interferon α + Fe-C | Standard of interferon α | Number of observations |
| $0.64x10^{-6}$ | 2.03±0.053* | 1.59±0.231 | n=9 |
| $1.28x10^{-6}$ | 1.33±0.128** | 0.905±0.088 | n=8 |
| *-P<0.1<br>**-P<0.05 | | | |

The results obtained indicate that the coordination compound increases the antiviral activity of interferon α in a certain range of its concentrations and provided it is introduced after it. This character of the effect of the coordination compound is due to the fact that when it is introduced before or together with the interferon, the culture medium does not contain oxidizing agent or it contains a relatively insufficient amount thereof. Owing to the complex composition of the culture medium, small amounts of coordination compound disappear relatively quickly from the culture medium into the intracellular space. The action of interferon on tropic cells is accompanied by production of oxidizing agents, but the time taken for them to be produced in sufficient quantity exceeds the residence time of the coordination compound in the culture medium. Preliminary introduction of interferon and its action on the tropic cells promote production of oxidizing agent by these cells, and this is used subsequently in catalytic action on the sulfhydryl groups of various receptors, including interferon receptors, which ultimately also promotes an increase in the number of cells interacting with the interferon, and this in its turn predetermines intensification of its antiviral effect. Thus, coordination compounds are able to increase the effectiveness of the action of cytokines on cells, increasing the number of cells capable of receptor-mediated interaction with cytokines.

Example 12. Influence of a coordination compound on the antiviral efficacy of Zovirax

Purpose of the work. To investigate the influence of a coordination compound on the therapeutic efficacy of an antiviral preparation.

The test compounds used were a coordination compound of cysteine with iron (Fe-C), which was introduced in an excess of cysteine in the ratio 1:1000 (Fe-C-:C), and the antiviral preparation Zovirax.

The study was conducted on a model of generalized herpetic infection of noninbred white mice - males weighing 16-18 g, which were infected by the causative agents of herpes type 2 (HSV-2), which causes viral infection of urogenital localization in humans.

The infectious material (brain from suckling mice infected with HSV-2) was triturated in a mortar in the cold, a 10% suspension was prepared, centrifuged at +4°C (2000 rev/min) for 20 min, and then 10-fold dilutions were prepared. The white mice were infected intraperitoneally.

For carrying out the experimental investigations, the animals were divided into 8 groups:

Group No. 1 - virus control. The animals were infected with HSV-2 virus; the animals did not receive any injections;
Group No. 2 - solvent control. The animals were infected with HSV-2 virus and then 0.5 ml physiological solution was administered intraperitoneally for 14 days;
Group No. 3 - the animals were administered, daily for 14 days, the coordination compound at a dose of 20.0 mg/kg/0.5 ml (amount of coordination compound $1.7\times10^{-7}$M/kg); after infection with HSV-2 virus;
Group No. 4 - the animals were administered, daily for 14 days, the compound cysteine at a dose of 20.0 mg/kg/0.5 ml after infection with HSV-2 virus;
Group No. 5 - the animals were administered, daily for 14 days, the antiviral preparation Zovirax at a dose of 30.0 mg/kg/0.5 ml after infection with HSV-2 virus;
Group No. 6 - the animals were administered, daily for 14 days, the antiviral preparation Zovirax at a dose of 15.0 mg/kg/0.5 ml after infection with HSV-2 virus;

Group No. 7 - after infection with HSV-2 virus, the animals were administered, daily for 14 days, the antiviral preparation Zovirax at a dose of 15.0 mg/kg and the coordination compound Fe-C-:C - 20.0 mg/kg which were applied in 0.5 ml (amount of coordination compound $1.7 \times 10^{-7}$M/kg);

Group No. 8 - after infection with HSV-2 virus, the animals were administered, daily for 14 days, the antiviral preparation Zovirax at a dose of 15.0 mg/kg and cysteine - 20.0 mg/kg which were applied in 0.5 ml.

The efficacy of the preparations was assessed on the basis of 3 indices:
a) lethality, b) percentage protection (difference in survival rate in the virus experiment and control), c) mean life (ML). The results obtained are shown in Table 7

**Table 7** Influence of the coordination compound on the efficacy of antiviral therapy with the preparation Zovirax

| Grou p No. | Preparations | Died/total in group | % mortality | % protection (S) | ML (days) (T) |
|---|---|---|---|---|---|
| 1. | Control (untreated) | 10/16 | 62.5 | - | 9.25 |
| 2. | Control (physiolog. solution 0.5 ml s/c) | 10/16 | 62.5 | - | 9.25 |
| 3. | Treatment: Fe-C - 20 mg/kg | 8/16 | 50 | 12.5 | 12.8 |
| 4. | Treatment: cysteine 20 mg/kg | 10/16 | 62.5 | - | 11.4 |
| 5. | Treatment: Zovirax 30 mg/kg | 0/16 | 0 | 62.5 | - |
| 6. | Treatment: Zovirax 15 mg/kg | 8/16 | 50 | 12.5 | 12.8 |
| 7. | Treatment: Fe-C - 20 mg/kg + Zovirax 15 mg/kg | 0/16 | 0 | 62.5 | - |
| 8. | Treatment: cysteine - 20 mg/kg + Zovirax 15 mg/kg | 8/16 | 50 | 12.5 | 12.8 |

It follows from the results obtained (Table 7) that with separate use of the test substances, the antiviral preparation Zovirax is an effective therapeutic agent when it is prescribed at a dose of 30 mg/kg (group 4) according to an indisputable criterion - the survival rate of the experimental animals. Reducing the dose of Zovirax to 15 mg/kg led to a decrease in efficacy of antiviral therapy (group 5) according to the criterion of survival rate. However, use of the antiviral preparation Zovirax at a dosage of 15 mg/kg in combination with the Fe-C coordination compound promoted an increase in therapeutic efficacy of Zovirax. Without the coordination compound, cysteine was unable to intensify the antiviral activity of the preparation.

Thus, the data obtained indicate that nano-amounts of a coordination compound based on a d-metal (about 200nM/kg), administered with an agent for etiotropic antiviral therapy of herpetic infection, are able to increase its therapeutic efficacy. This effect may be due to the ability of the coordination compound to increase the sensitivity of virus-infected animal cells to the action of endogenous interferon $\alpha$, which in combined use with an antiviral agent was manifested by an increase in its therapeutic efficacy, i.e. decrease in the dose of Zovirax required to achieve the necessary therapeutic effect.

<u>Example 13. Influence of a coordination compound on the antibacterial efficacy of josamycin in experimental generalized chlamydial infection</u>

Purpose of the work. To investigate the influence of a coordination compound on the therapeutic efficacy of an antibiotic.

The test compounds used were a coordination compound of cysteine with iron (Fe-C), which was introduced in an excess of cysteine in a ratio of 1:1000 (Fe-C-:C), and the antiviral preparation josamycin (Wilprafen). The experiment was carried out on noninbred male white mice weighing 17-19 g. The animals were divided into the following groups with 16 animals in each.

1. Control (untreated);
2. Control (physiolog. solution 0.2 ml s/c);
3. Treatment: Fe-C-:C - 20 mg/kg (amount of coordination compound $1.7x10^{-7}$M/kg);
4. Treatment: cysteine 20 mg/kg;
5. Treatment: josamycin 25 mg/kg;
6. Treatment: josamycin 50 mg/kg;
7. Treatment: josamycin 100 mg/kg;
8. Treatment: josamycin 25 mg/kg + Fe-C-:C 20 mg/kg (amount of coordination compound $1.7\times10^{-7}$M/kg);
9. Treatment: josamycin 50 mg/kg+ Fe-C-:C 20 mg/kg (amount of coordination compound $1.7\times10^{-7}$M/kg);
10. Treatment: josamycin 25 mg/kg + cysteine 20 mg/kg;
11. Treatment: josamycin 50 mg/kg +cysteine 20 mg/kg.

The animals were infected with the pathogen Chlamydia trachomatis, strain L-2, which causes urogenital chlamydiosis in humans.

The infectious material (yolk sacs of chick embryos that have developed for 7 days) was applied to the mice intraperitoneally in a volume of 0.5 ml at a calculated dose of 2 $LD_{50}$.

Treatment was started 2 h after infection. Then the preparations were administered daily, once a day for 10 days. The observation time was 3.5 weeks.

Route of administration of the preparations: *subcutaneously* - cysteine and coordination compound of cysteine with iron (Fe-C), which was introduced in an excess of cysteine in the ratio 1:1000 (Fe-C-:C), physiological solution; *orally* - josamycin.

The preparations were administered in a volume of 0.5 ml daily for 10 days. The efficacy of the preparations was assessed from 3 indices: a) lethality, b) percentage protection (difference in survival rate in experiment and control of the causative agent), c) harmonic mean life (HML).

The experimental groups, the doses of the test preparations and the results obtained are presented in Table 8

Table 8. Influence of the coordination compound on the efficacy of antibacterial therapy with the preparation josamycin

| Group No. | Preparations | Died/tot al in group | % mortality | % protection , (S) | HML (T), (days) |
|---|---|---|---|---|---|
| 1. | Control (untreated) | 12/16 | 75 | 0 | 3.85 |
| 2. | Control (physiolog. solution 0.2 ml s/c) | 12/16 | 75 | 0 | 5.6 |
| 3. | Treatment: Fe-C - 20 mg/kg | 10/16 | 62.5 | 12.5 | 7.8 |
| 4. | Treatment: cysteine 20 mg/kg | 12/16 | 75 | 0 | 5.6 |
| 5. | Treatment: josamycin 25 mg/kg | 6/16 | 37.5 | 37.5 | 12.35 |
| 6. | Treatment: josamycin 50 mg/kg | 2/16 | 12.5 | 62.5 | 83.3 |
| 7. | Treatment: josamycin 100 mg/kg | 0/16 | 0 | 75 | ∞ |
| 8. | Treatment: josamycin 25 mg/kg + Fe-C 20 mg/kg | 4/16 | 25 | 50 | 25.6 |

(continued)

| Group No. | Preparations | Died/tot al in group | % mortality | % protection , (S) | HML (T), (days) |
|---|---|---|---|---|---|
| 9. | Treatment: josamycin 50 mg/kg + Fe-C 20 mg/kg | 0/16 | 0 | 75 | ∞ |
| 10. | Treatment: josamycin 25 mg/kg + cysteine 20 mg/kg | 6/16 | 37.5 | 37.5 | 7.4 |
| 11. | Treatment: josamycin 50 mg/kg + cysteine 20 mg/kg | 4/16 | 25 | 50 | 25.6 |

It follows from the results obtained (Table 8) that with separate use of the test substances, josamycin is an effective therapeutic agent when prescribed at a dose of 100 mg/kg (group 7) according to the indisputable criterion of the survival rate of the experimental animals. Reducing the dose of josamycin to 25 and 50 mg/kg led to a decrease in efficacy of antibacterial therapy (groups 5 and 6) according to the criterion of survival rate. However, use of josamycin in dosages of 25 and 50 mg/kg in combination with the Fe-C coordination compound (amount of coordination compound $1.7\times10^{-7}$M/kg) promoted an increase in therapeutic efficacy of the preparation. It follows from the experimental results that the coordination compound made it possible to halve the therapeutic dose of the preparation without loss of therapeutic efficacy. Drug resistance of chlamydia to antibacterial preparations is due to their capacity for intracellular parasitization. Chlamydia can be detected in submembrane vesicles of affected cells, obtaining their necessary nutrients from the cytosol of the cells. With respect to antibacterial agents, the chlamydia are in a pharmacological refuge [4]. Receptor-mediated action of coordination compounds in combination with a labile ligand possibly stimulates the secretion of submembrane vesicles with the pathogen. This assertion is supported by the ability of coordination compounds to increase the amount of the intracellular ionized form of calcium that is sufficient for effecting said action.

Thus, the data obtained point to the ability of nano-amounts of a coordination compound based on a d-metal (about 200nM/kg), administered with an agent for etiotropic antibacterial therapy of chlamydial infection, to increase its therapeutic efficacy.

The results in examples 10-13 show that coordination compounds at concentrations that are characteristic of the action of bioregulators (cytokines, hormones) and other biologically active substances (alkaloids, vitamins etc.), exert an influence on the therapeutic efficacy of pharmaceutical preparations, which is reflected in achievement of therapeutic action of the pharmaceutical preparation at lower single doses and overall doses than are required for achievement of the required therapeutic effect in the absence of the coordination compound. Achievement of the therapeutic effect at lower single and overall doses and/or decrease in toxicity of medicinal products permit fuller utilization of the therapeutic potential of the medicinal product, which may moreover be a ligand, an inorganic or organic counterion of a ligand.

The results obtained (examples 7-13) indicate that small and ultra-small amounts of coordination compounds of d-metals possess intrinsic biological activity, which can be assessed quantitatively both from their action on cultured cells, and when administered to a patient. The experimental data indicate that the molecular target of the small and ultra-small amounts of coordination compounds of d-metals in the cell are the cell-surface receptor, ion channels, key intracellular enzymes and other biological molecules that regulate cellular functions. Variation of the molecular targets of coordination compounds is transformed into various biochemical reactions, for example expression of the enzymes of two-phase detoxication of xenobiotics, calcium-mediated processes, sensitivity of the cells to the action of interferon, which in its turn predetermines the variation in activity of various cellular processes. With reference to the results in these examples, it is the ability of the cells to withstand the negative action of physical, chemical and biological factors, increase the efficacy of pharmacotherapy with cytokines, antibiotics, and antiviral agents. This kind of action of chemical compounds on cells and on the organism as a whole, and on their interactions with other pharmaceutical preparations is of interest for practical pharmacology and pharmacotherapy, making it possible to create novel medicinal products for low-dose therapy [4]. Creation of effective agents for low-dose therapy is urgent because mortality from side-effects of widely used medicinal products occupies fifth place in overall mortality [8]. The approach proposed by the invention makes it possible to reduce or eliminate the toxic side-effects of pharmaceutical preparations on cells, tissues or organs

that are not affected by the disease, but owing to the pharmacokinetics and pharmacodynamics of the medicinal product constitute a target of its action. For example, agents for chemotherapy of malignant neoplasms should ideally act on tumor cells, antibiotics should only act on microorganisms, and antiviral preparations only on virus-infected cells. However, the pharmaceutical preparations in the aforementioned pharmacological groups, and others, do not possess this selectivity [4].

An experimentally proven property of affected cells (e.g. tumor cells, virus-infected cells, those affected by a microorganism and the microorganism itself) is drug resistance, in contrast to its absence in unaffected cells [4]. In this connection, the range of protective reactions that can be induced by small and ultra-small amounts of coordination compounds of d-metals makes it possible above all to reduce the negative effects of chemotherapy on normal cells. The capacity of small and ultra-small amounts of coordination compounds of d-metals to increase the efficacy of the action of a medicinal product on a patient's body, which is reflected in a decrease in dose of the medicinal product necessary for achieving the desired therapeutic result, means that coordination compounds can be regarded as promising agents for designing preparations for low-dose therapy.

Industrial applicability

Thus, the invention makes it possible to use small and ultra-small amounts of coordination compounds of d-metals for pharmacological solutions in the creation of pharmaceutical preparations based on them in accordance with the biological activity and pharmacological action and use of coordination compounds of d-metals for enhancing the pharmacological activity and/or lowering the toxicity of the active principle of pharmacopeial medicinal products.

Design of pharmaceutical preparations based on small and ultra-small amounts of coordination compounds of d-metals, their combined use for creating pharmacological substances and dosage forms with active principles of pharmacopeial medicinal products, which can be selected so that their pharmacological effects correspond to the pharmacological effects of a medicinal product or reduce its toxic effects on a patient's body, is illustrated in Fig. 11.

REFERENCES

1. Basolo F., Pearson R. Mechanisms of inorganic reactions. Investigation of complexes of metals in solution. Moscow: Mir. 1971. 592 p.
2. Tolstikova T.G. et al. On the road to low-dose medicines / Vestnik rossiiskoi akademii nauk. 2007. Vol. 77. No. 10. p.867-874.
3. Alonso-Torre S.R., Trautmann A. 1993. Calcium responses elicited by nucleotides in macrophages. Interaction between two receptor subtypes. J. Biol. Chem. 268: 18640-18647.
4. Basic & Clinical Pharmacology. Edited by Bertram G. Katzung, MD, PhD, 1995.
5. Conrad R.E. 1981. Induction and collection of peritoneal exudate macrophages. Manual of macrophages methodology. New York: Marcell Dekker. P. 5-11
6. Grynkiewicz G., Poenie M., Tsien R.Y. 1985. A new generation of Ca2+ indicators with greatly improved fluorescence properties. J. Biol. Chem. 260: 3440 - 3450.
7. Monahan R.A., Dvorak H.F., Dvorak A.M. 1981. Ultrastructural localization of nonspecific esterase activity in guinea pig and human monocytes, macrophages and lymphocytes. Blood. 1981. 58: 1089-1099.
8. Pirmohamed M., Kevin B. Genetic susceptibility to adverse drug reactions//Trends Pharm. Sci. 2001. V. 22. P. 298-305.
9. Randriamampita C., Trautmann A. 1987. Ion channels in murine macrophages. Cell Biol. 105: 761-769.
10. Spiccia L., Inorg. Chim. Acta. 2004. V. 357. P. 2799.
11. Junk P.C., McCool B.J., Moubaraki B. et al. // Angewandte Chemie. Int. Ed. Engl. 1999. V. 38. P. 2224.


## Claims

**1.** A composition containing, in aqueous solution, bi- or oligonuclear coordination compounds, formed by identical or different atoms of d-metals, joined together by one or more bridging groups, and containing, in the composition of the inner sphere of each d-metal atom, water molecules and/or hydroxide groups, as well as labile ligands, which are easily replaced with sulfur atoms of thioamino acids or peptides, such as mono-, bi- or higher-dentate organic molecules with bridging and nonbridging types of coordination to the atoms of d-metals.

**2.** The composition as claimed in claim 1, **characterized in that** the atoms of a d-metal (d-metals) in the coordination

compound are joined together by one or more bridging oxo and/or hydroxo groups.

3. The composition as claimed in claim 1, containing, in aqueous solution, binuclear coordination compounds formed by d-metal atoms joined together by one or more bridging groups, and containing, in the composition of the inner sphere of each d-metal atom, water molecules and/or hydroxide groups, as well as labile ligands, which can easily be substituted for sulfur atoms of thioamino acids or peptides.

4. The composition as claimed in claim 3, **characterized in that** said binuclear compound has a framework selected from the group comprising frameworks of the following formulas:

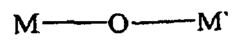

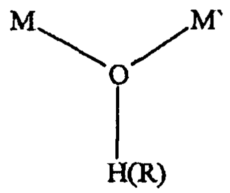

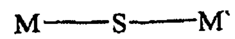

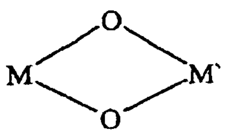

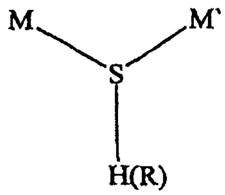

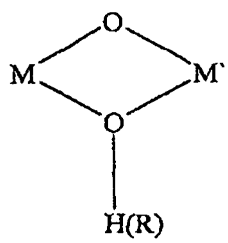

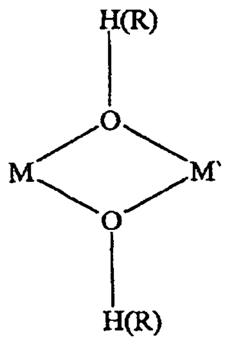

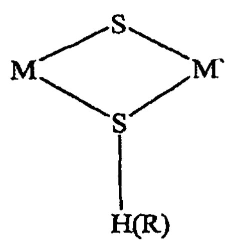

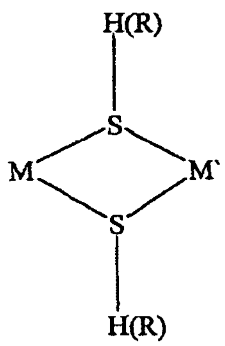

5. The composition as claimed in claim 1, containing, in the composition of the inner sphere of each d-metal atom, strongly bound ligands of any nature and coordination number with bridging and nonbridging types of coordination to the atoms of d-metals.

6. The composition as claimed in claim 1, **characterized in that** the labile ligands are oxygen-containing and/or nitrogen-containing and/or sulfur-containing organic molecules.

7. The composition as claimed in claim 1, **characterized in that** the concentration of d-metal (d-metals) in said aqueous

solution is less than $10^{-3}$ M.

**8.** The composition as claimed in claim 1, also comprising free molecules of the compound, by which said labile ligand is represented, not coordinated to the d-metal atoms, **characterized in that** the molar ratio atom of d-metal (d-metals): molecule of compound of the labile ligand is from 1:1 to 1:30000.

**9.** The composition as claimed in claim 1, **characterized in that** the coordination compound is obtained by interaction of aqua-hydroxo complexes of a d-metal (d-metals) and solutions of said labile ligands.

**10.** The composition as claimed in claim 1, which increases the therapeutic efficacy of pharmacologically active substances.

**11.** The composition as claimed in claim 10, **characterized in that** said pharmacologically active substance is said labile ligand.

**12.** The composition as claimed in claim 10, **characterized in that** said pharmacologically active substance and said labile ligand are different substances.

**13.** The composition as claimed in claim 11, comprising free molecules of said pharmacologically active substance.

**14.** The composition as claimed in claim 10, **characterized in that** the pharmacodynamics and/or pharmacokinetics of the coordination compound correspond to that of said pharmacologically active substance.

**15.** The composition as claimed in claim 10, **characterized in that** the transition metal in the composition of the coordination compound is iron, copper, palladium or some other d-metal.

**16.** The composition as claimed in claim 15, **characterized in that** coordination compounds of various d-metals can be used.

**17.** The composition as claimed in claim 1, which is stored in dried form.

**18.** The composition as claimed in any one of claims 1-17, which exerts an action on the biological activity of cells by modulating the activity of extracellular, cell-surface and intracellular receptors, enzymes, ion channels, transporter proteins of cytoplasmic and intracellular membranes, and extracellular regulatory and transport molecules of a peptide nature.

**19.** The composition as claimed in any one of claims 1-17, possessing specific cellular tropism and/or organotropism and/or specific systemic action and at least one pharmacological activity, selected from the group comprising antiviral, antibacterial, antiproliferative, proapoptotic, cytoprotective, direct and indirect antioxidant, hemostimulating, antihypoxanthine, immunomodulating, toxicity-modifying, antifibrotic, antiinflammatory activity as well as ability to suppress reactions of multiple drug resistance of various genesis, modulate regulatory, metabolic, and immune dysfunctions and imbalances, which can be used for production of a medicinal product for external, inhalational, enteral and parenteral application.

**20.** A method of therapeutic action on a patient's body, **characterized in that** a patient requiring this is administered an effective amount of the composition as claimed in claims 1-19.

**21.** A method of increasing the therapeutic efficacy of a pharmacologically active substance, **characterized in that** the patient is administered the composition as claimed in claims 1-19 simultaneously or successively with said substance.

**22.** The method as claimed in claim 21, **characterized in that** said pharmacologically active substance and the composition as claimed in claims 1-9 are administered in the composition of one dosage form.

**23.** The method as claimed in claim 20 or 21, **characterized in that** the amount of coordination compound of a d-metal (d-metals) administered to the patient is $10^{-3}$ - $10^{-12}$ mol/kg of body weight.

**24.** The method as claimed in claim 20 or 21, **characterized in that** the mass of d-metal (d-metals) administered in the composition of the coordination compound does not exceed the physiologically permissible value for the metal.

**25.** The method as claimed in claim 20 or 21, **characterized in that** the effect is achieved at low or ultra-low concentrations of the composition as claimed in claim 1-19, applied externally or administered to a patient by inhalation or by the enteral or parenteral route.

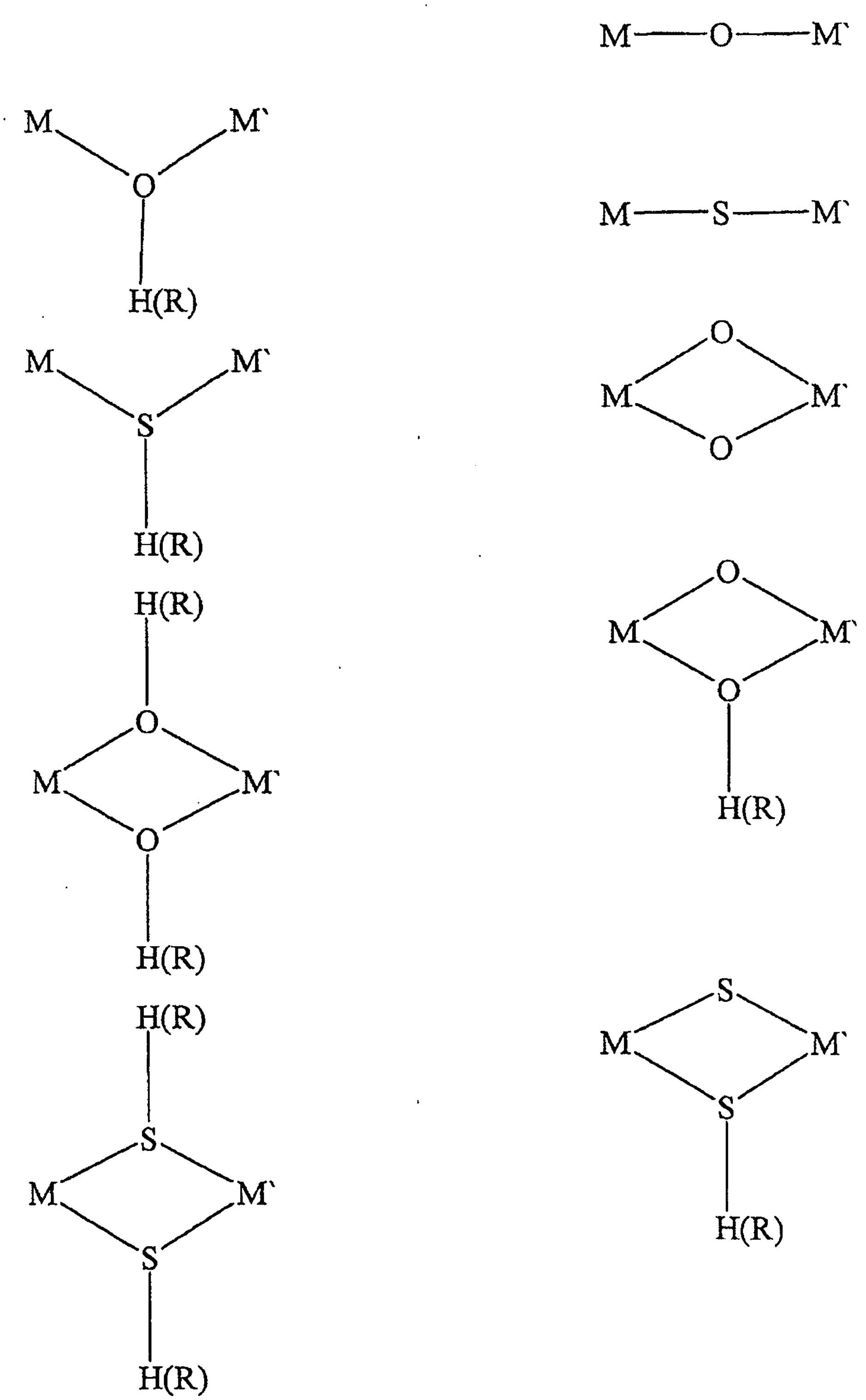

Fig. 1

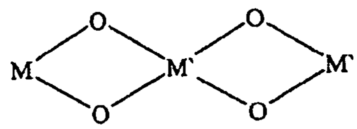

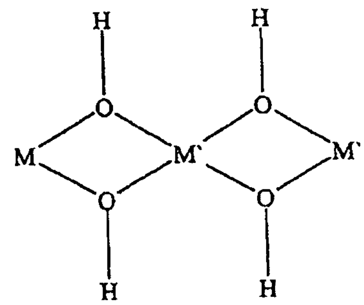

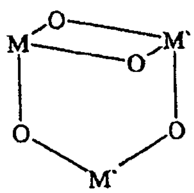

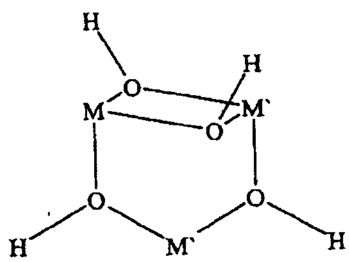

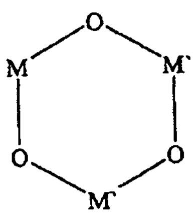

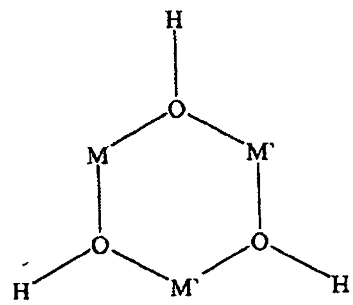

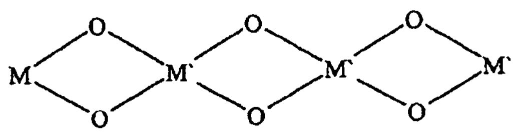

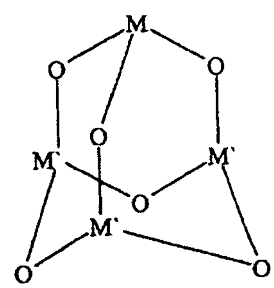

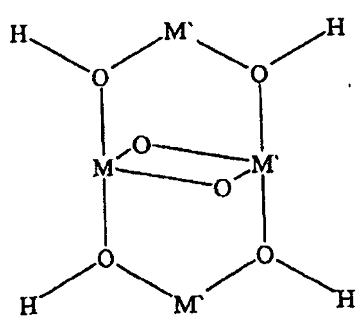

Fig. 2

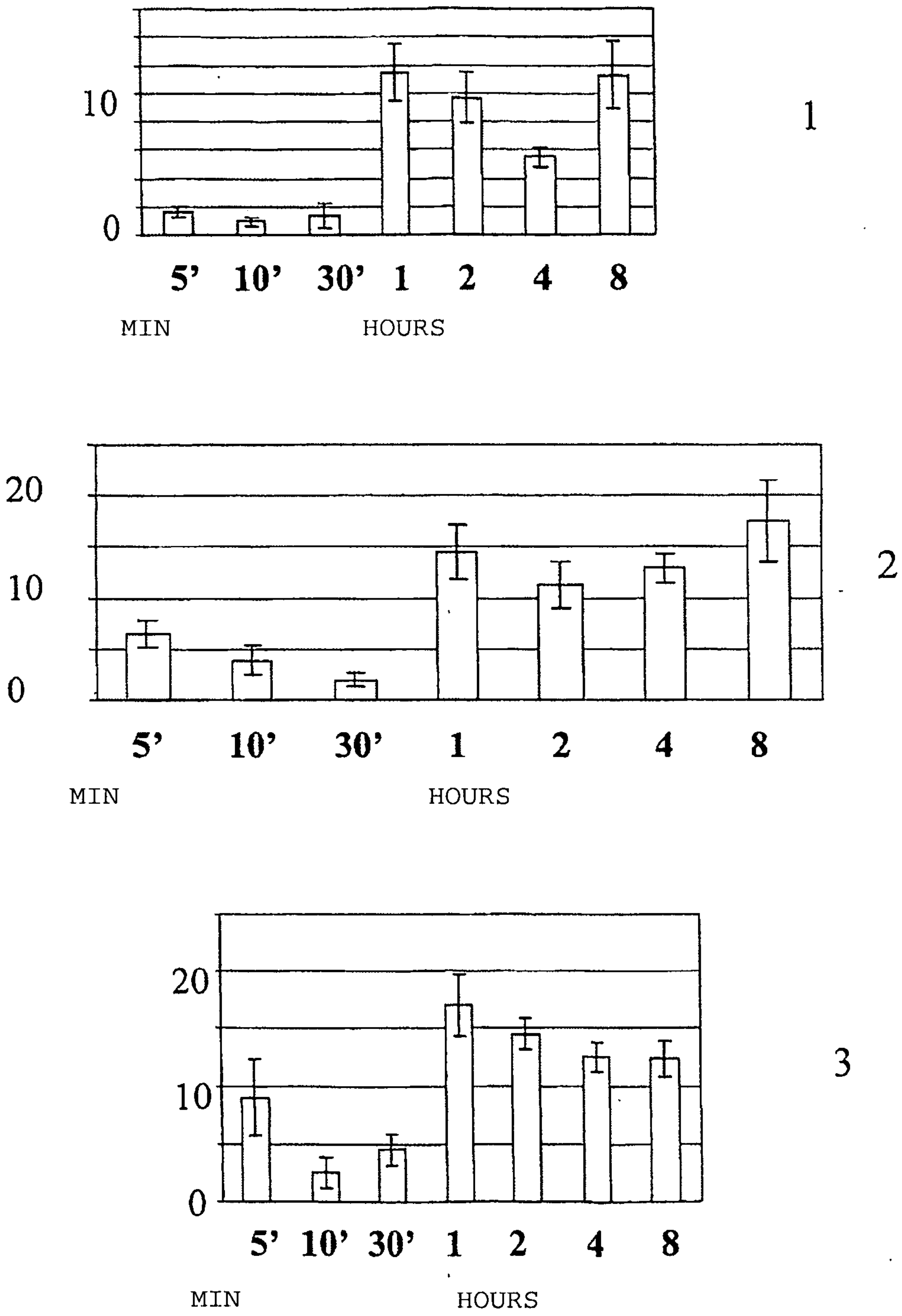
10
0
5' 10' 30' 1 2 4 8
MIN
HOURS
1
20
10
0
5' 10' 30' 1 2 4 8
MIN
HOURS
2
20
10
0
5' 10' 30' 1 2 4 8
MIN
HOURS
3

Fig. 3

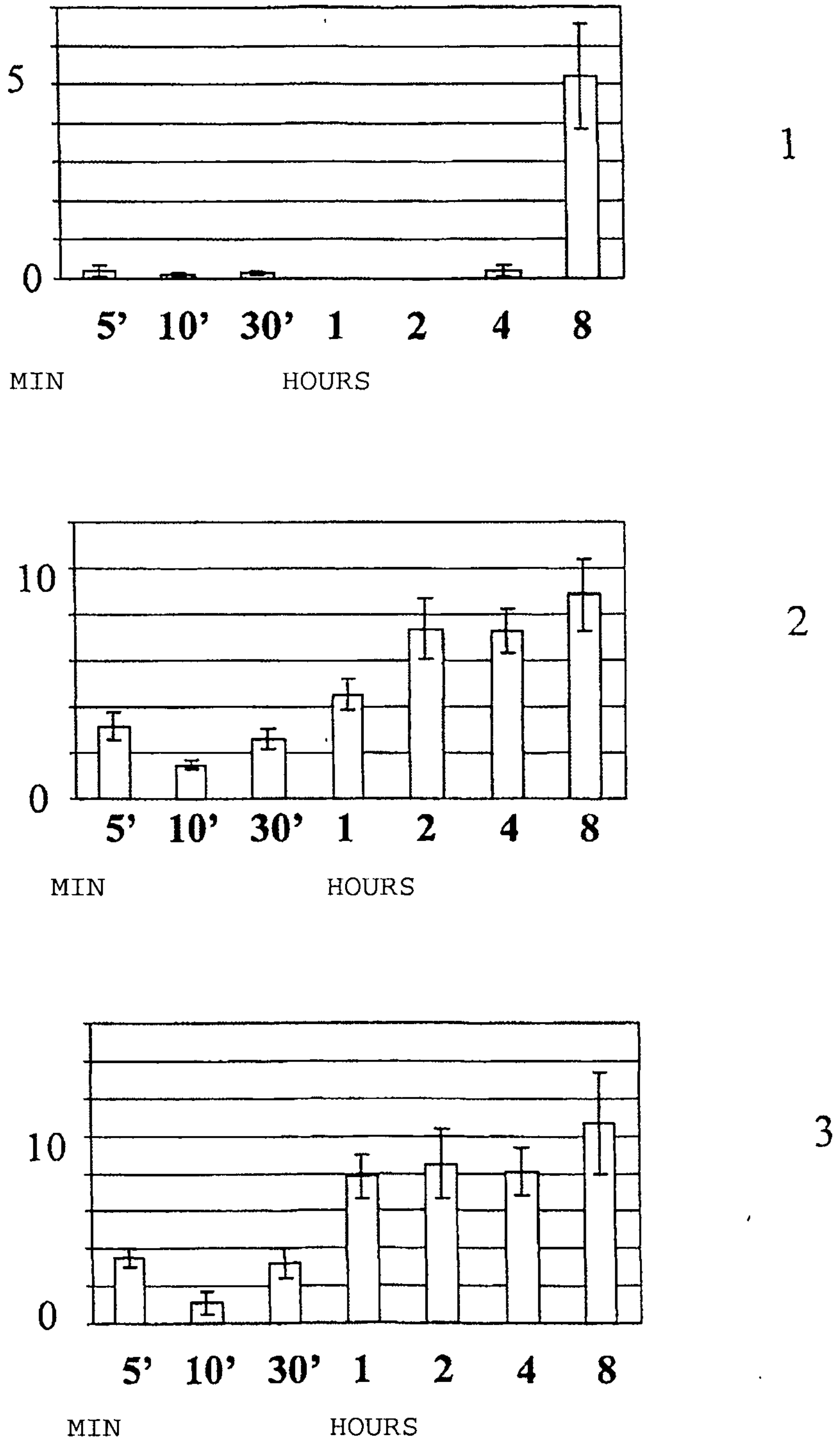

5
0
5' 10' 30' 1 2 4 8
MIN HOURS
1
10
0
5' 10' 30' 1 2 4 8
MIN HOURS
2
10
0
5' 10' 30' 1 2 4 8
MIN HOURS
3


Fig. 4

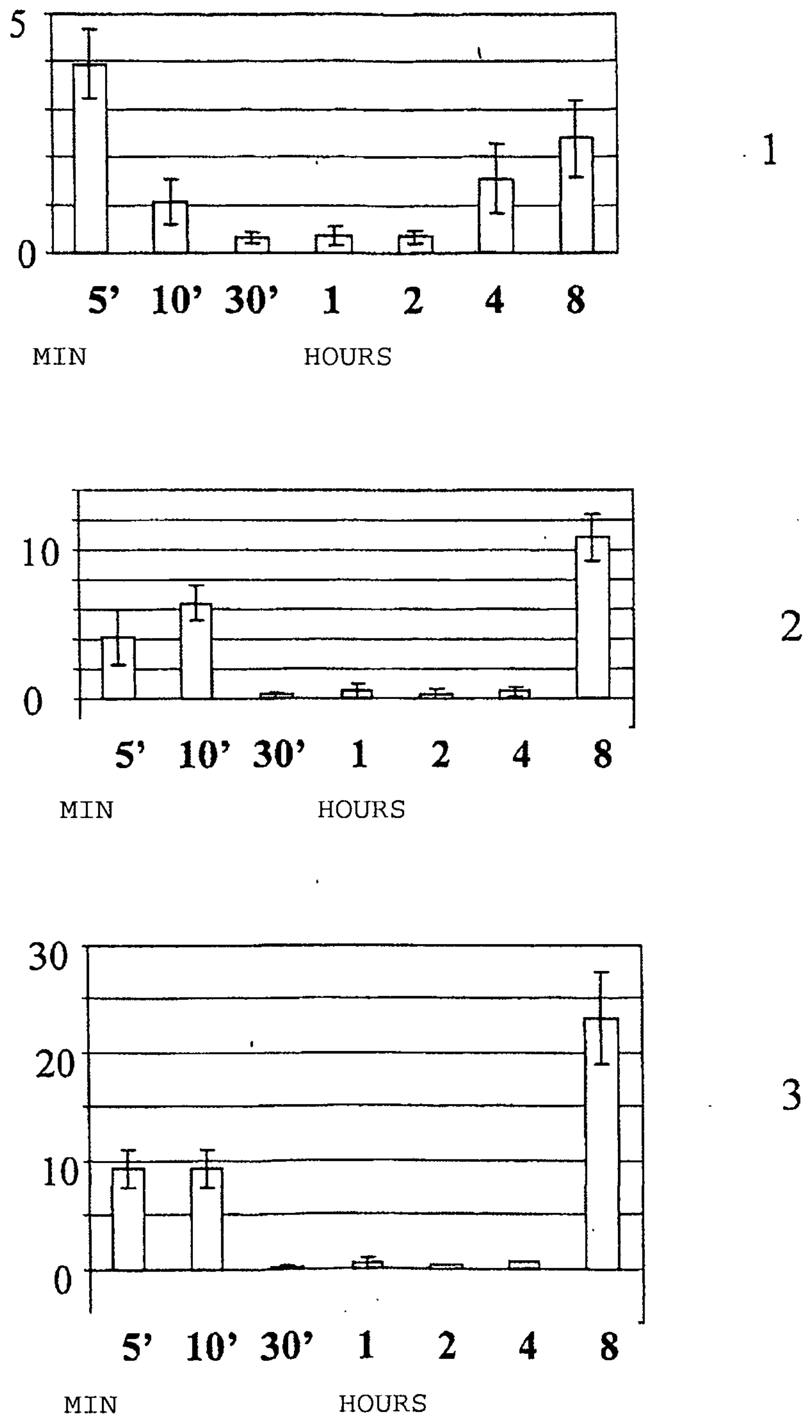
5
0
5' 10' 30' 1 2 4 8
MIN
HOURS
1
10
0
5' 10' 30' 1 2 4 8
MIN
HOURS
2
30
20
10
0
5' 10' 30' 1 2 4 8
MIN
HOURS
3

Fig. 5

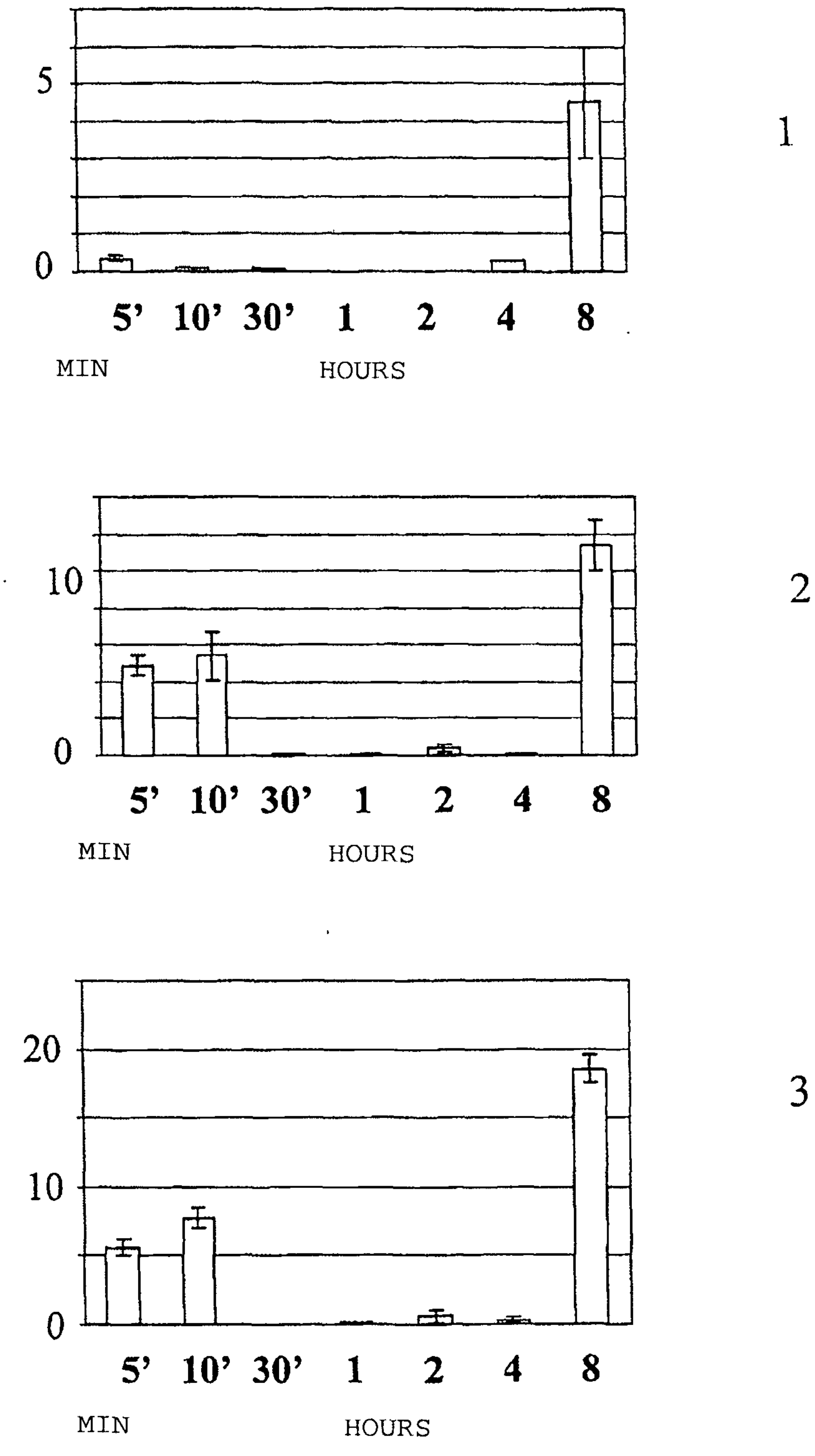
5
0
5' 10' 30' 1 2 4 8
MIN
HOURS
1
10
0
5' 10' 30' 1 2 4 8
MIN
HOURS
2
20
10
0
5' 10' 30' 1 2 4 8
MIN
HOURS
3

Fig. 6

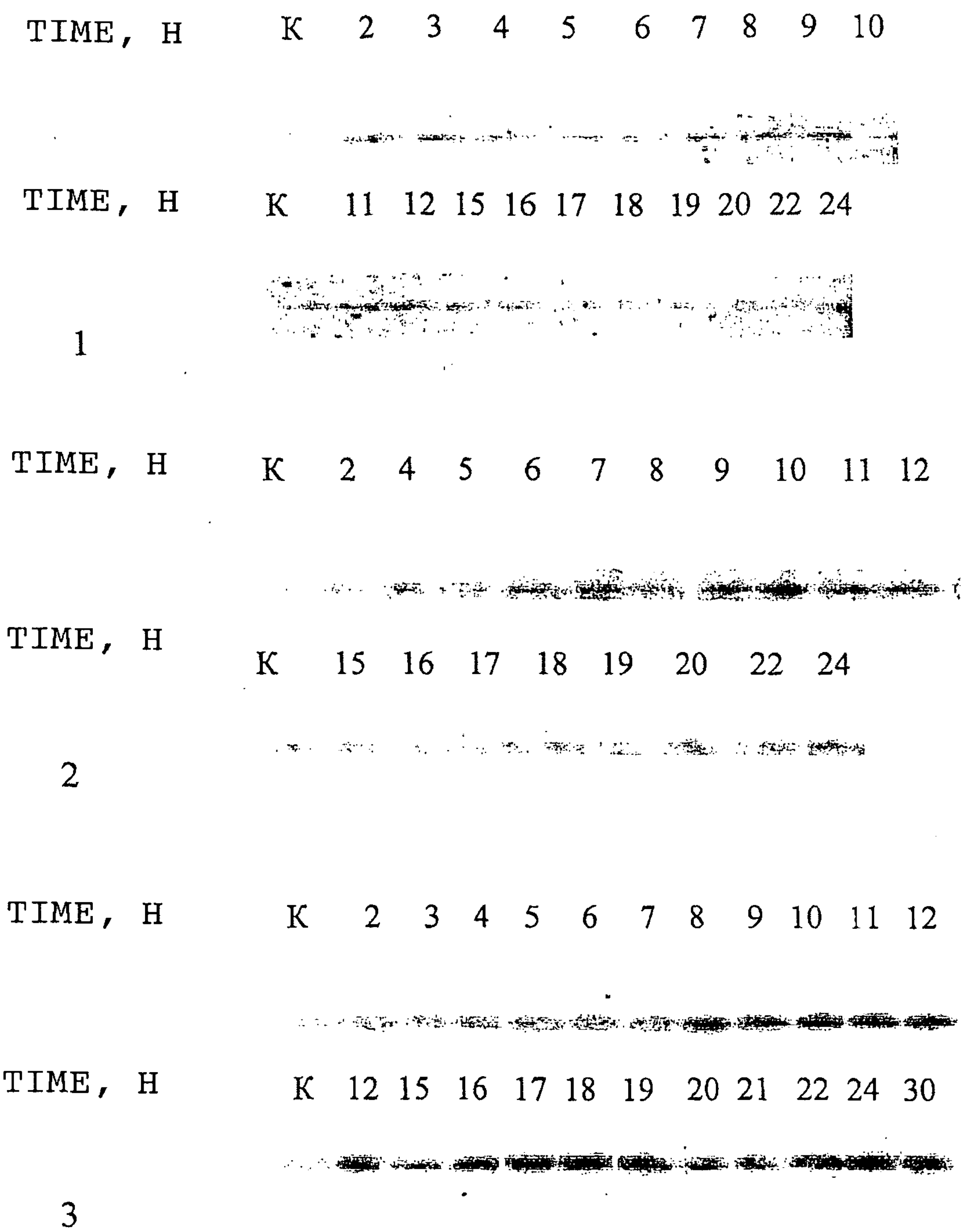
TIME, H
K 2 3 4 5 6 7 8 9 10
TIME, H
K 11 12 15 16 17 18 19 20 22 24
1
TIME, H
K 2 4 5 6 7 8 9 10 11 12
TIME, H
K 15 16 17 18 19 20 22 24
2
TIME, H
K 2 3 4 5 6 7 8 9 10 11 12
TIME, H
K 12 15 16 17 18 19 20 21 22 24 30
3

Fig. 7

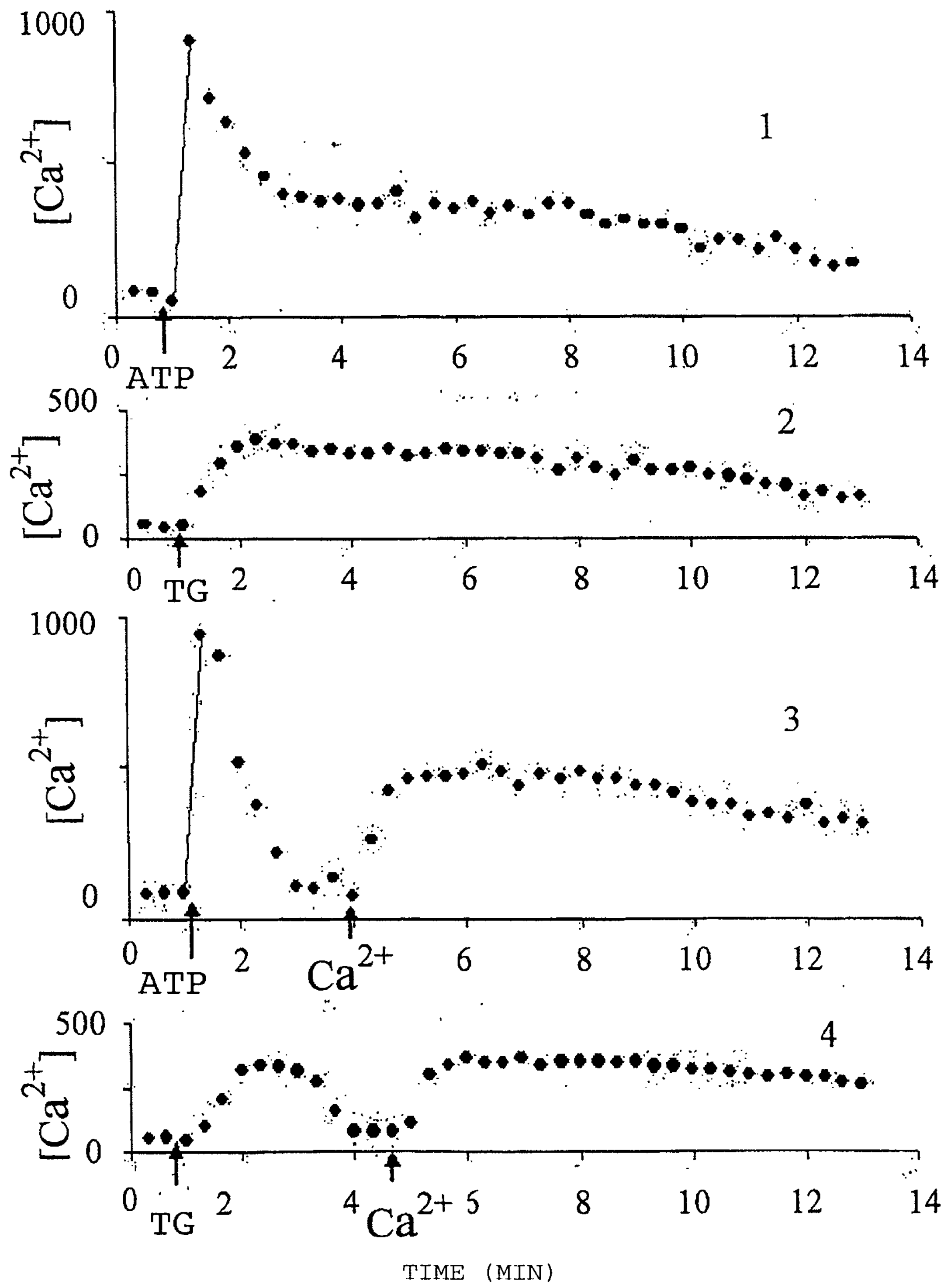
1000
[Ca2+]
0
1
0
ATP
2
4
6
8
10
12
14
500
[Ca2+]
0
2
TG
1000
[Ca2+]
3
Ca2+
500
4
TIME (MIN)

Fig. 8

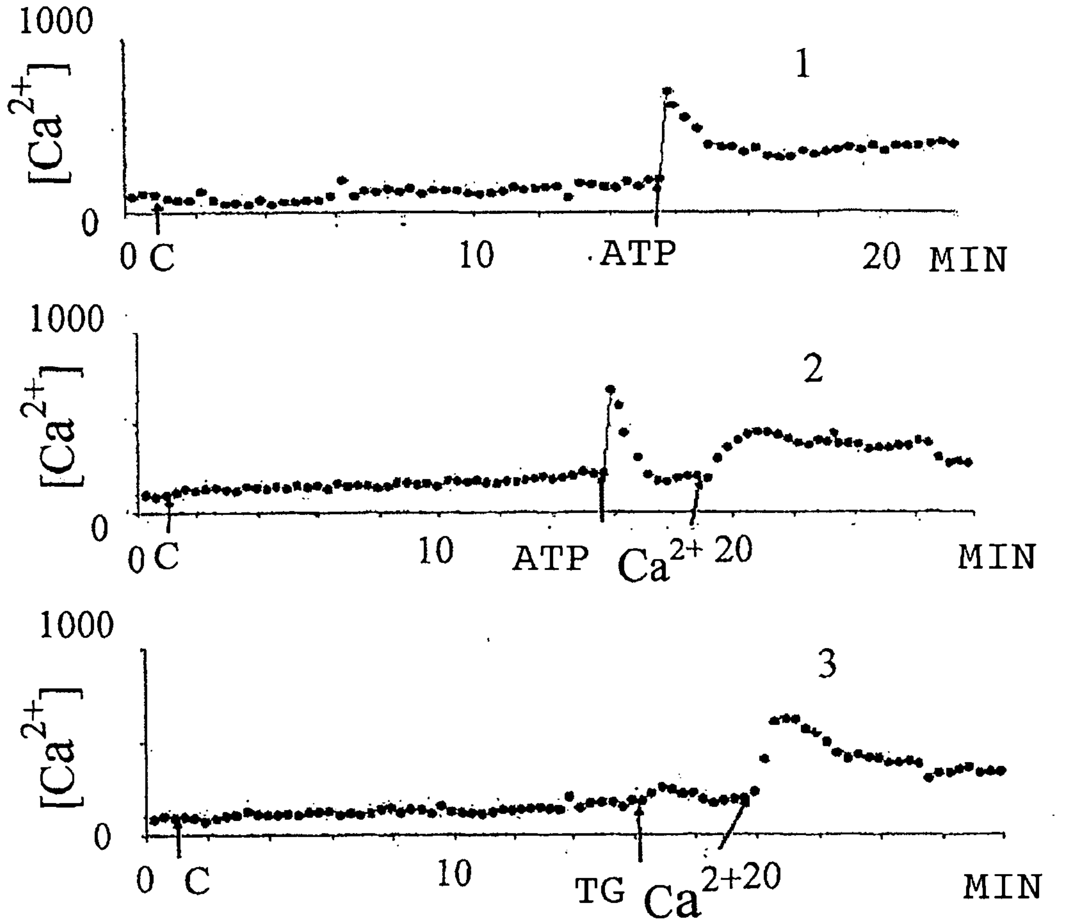
1000
[Ca2+]
0
1
0 C
10
ATP
20 MIN
1000
[Ca2+]
0
2
0 C
10
ATP
Ca2+
20
MIN
1000
[Ca2+]
0
3
0 C
10
TG
Ca2+
20
MIN

Fig. 9

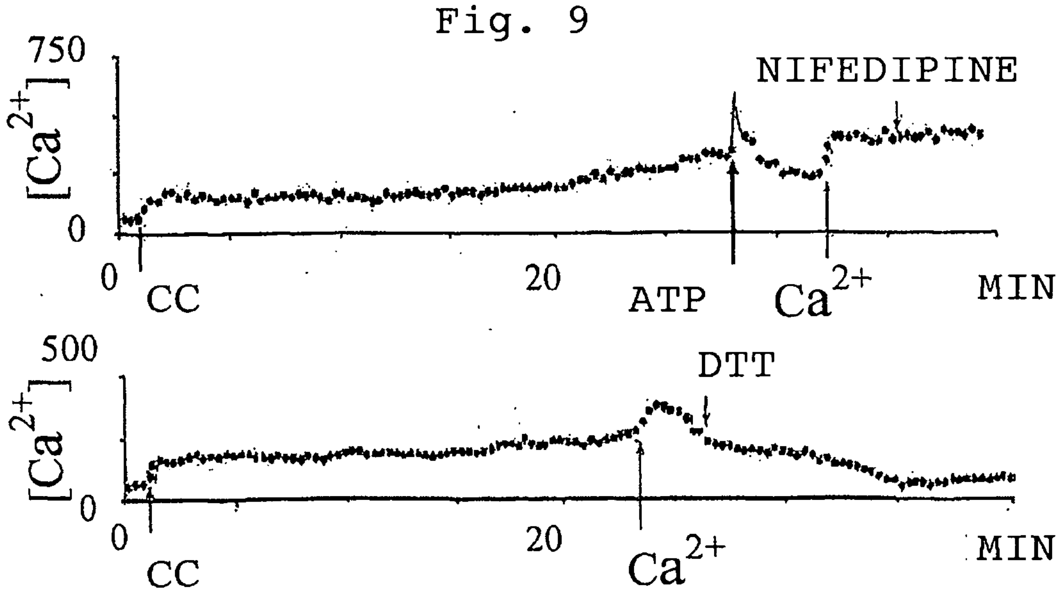
750
[Ca2+]
0
NIFEDIPINE
0
CC
20
ATP
Ca2+
MIN
500
[Ca2+]
0
DTT
0
CC
20
Ca2+
MIN

Fig. 10

PRIMARY ACTION OF
COORDINATION COMPOUNDS

- > Extracellular enzymes
- > Intracellular enzymes
- > Extracellular transporters
- > Membrane transporters
- > Cell-surface receptors
- > Intracellular receptors
- > Ion channels
- > Proteins of the cytoskeleton
- > ANTIGENS
- > Exocytosis
- > Endocytosis

Fig. 11

PHARMACOLOGICAL EFFECTS OF
COORDINATION COMPOUNDS

- IMMUNOMODULATING
- ANTIVIRAL
- ANTIBACTERIAL
- ANTIPROLIFERATIVE
- HEMOSTIMULATING
- ANTIOXIDANT
- WITH HYPOGLYCEMIC EFFECT
- OTHER PHARMACOLOGICAL EFFECTS

PHARMACOPEIAL
MEDICINAL PRODUCTS

IMMUNOMODULATING
ANTIVIRAL
ANTIBACTERIAL
ANTITUMOR
HEMOSTIMULATING
ANTIOXIDANT
WITH HYPOGLYCEMIC ACTION
OTHER PHARMACOPEIAL MEDICINAL PRODUCTS

MEDICINAL PRODUCTS BASED ON
COORDINATION COMPOUNDS

1

LOW-DOSE MEDICINAL PRODUCTS BASED
ON COORDINATION COMPOUNDS
in combination with combinable
pharmacological substances of
pharmacopeial medicinal products
2

Fig. 11

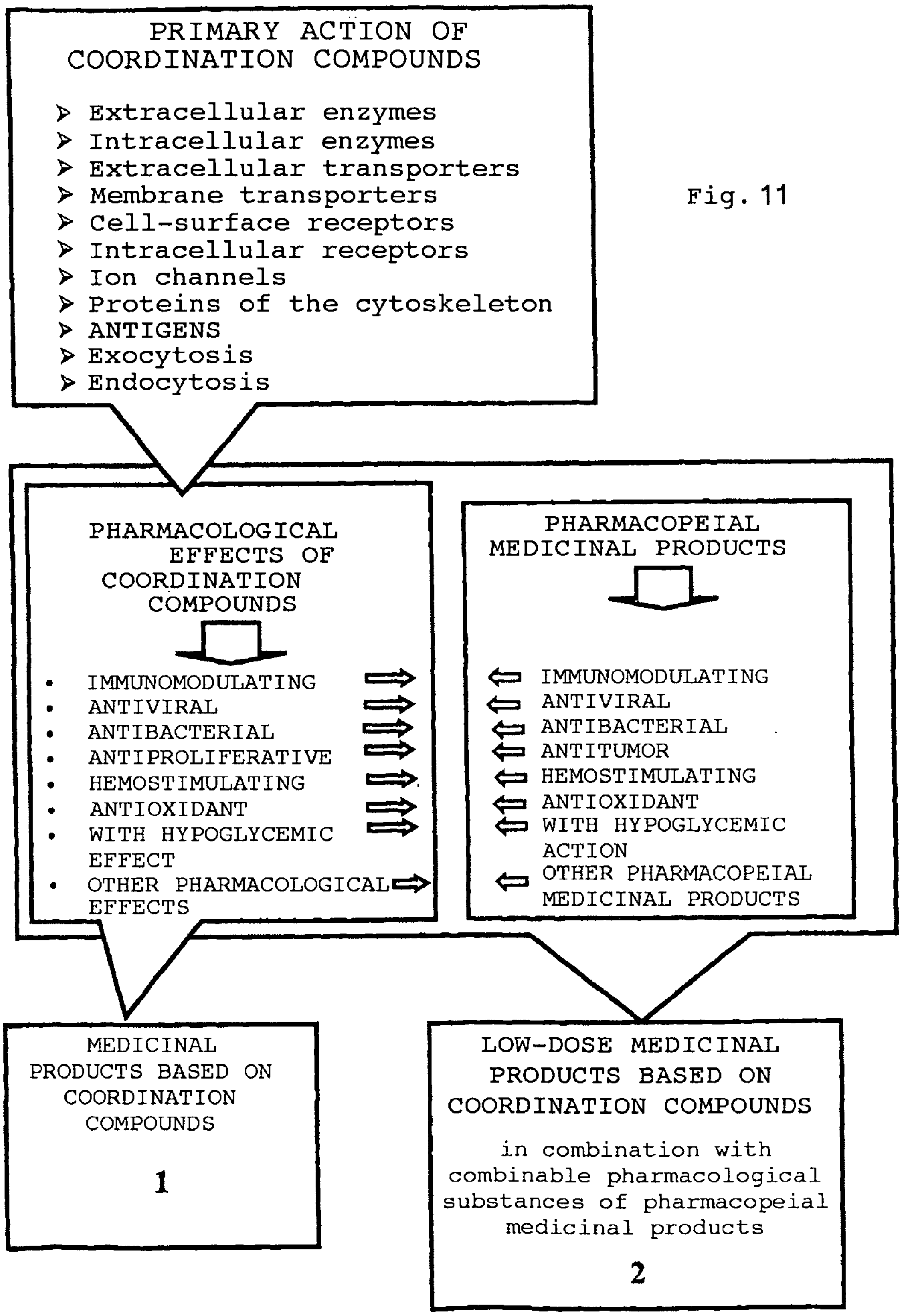
PRIMARY ACTION OF COORDINATION COMPOUNDS
Extracellular enzymes
Intracellular enzymes
Extracellular transporters
Membrane transporters
Cell-surface receptors
Intracellular receptors
Ion channels
Proteins of the cytoskeleton
ANTIGENS
Exocytosis
Endocytosis
PHARMACOLOGICAL EFFECTS OF COORDINATION COMPOUNDS
IMMUNOMODULATING
ANTIVIRAL
ANTIBACTERIAL
ANTIPROLIFERATIVE
HEMOSTIMULATING
ANTIOXIDANT
WITH HYPOGLYCEMIC EFFECT
OTHER PHARMACOLOGICAL EFFECTS
PHARMACOPEIAL MEDICINAL PRODUCTS
IMMUNOMODULATING
ANTIVIRAL
ANTIBACTERIAL
ANTITUMOR
HEMOSTIMULATING
ANTIOXIDANT
WITH HYPOGLYCEMIC ACTION
OTHER PHARMACOPEIAL MEDICINAL PRODUCTS
MEDICINAL PRODUCTS BASED ON COORDINATION COMPOUNDS
1
LOW-DOSE MEDICINAL PRODUCTS BASED ON COORDINATION COMPOUNDS
in combination with combinable pharmacological substances of pharmacopeial medicinal products
2

**INTERNATIONAL SEARCH REPORT**

International application No.
PCT/RU2009/000070

A. CLASSIFICATION OF SUBJECT MATTER

***see continuation sheet**

According to International Patent Classification (IPC) or to both national classification and IPC

B. FIELDS SEARCHED

Minimum documentation searched (classification system followed by classification symbols)

A61K 9/08, 33/00, 33/24, 33/26, 33/34, 31/195, 31/198, 38/00, A61 P 37/00, 43/00, 31/28, 31/555, 31/7135

Documentation searched other than minimum documentation to the extent that such documents are included in the fields searched

Electronic data base consulted during the international search (name of data base and, where practicable, search terms used)

ChemIDplus Advanced, Esp@cenet, RUPTO, USPTO, WIPO, DB "Rossyskaya meditsina"

C. DOCUMENTS CONSIDERED TO BE RELEVANT

| Category* | Citation of document, with indication, where appropriate, of the relevant passages | Relevant to claim No. |
|---|---|---|
| Y | RU 2115657 C1 (SHISHKOV JURY IVANOVICH) 20.07.1998, the abstract, page 3, column 2-line 4, column 1, line 4, page 4, column 2, lines 5-25, lines 58-61, page 5, column 1, line 40-column 2, line 5, page 6, column 2, line 61 -page 7, column 1, line 37, page 8, column 1, lines 21-53, the claims | 1-25 |
| Y | TABASSUM Sartaj et al. Novel bimetallic complexes of copper, nickel and manganese derived from the cobalt(III) complex and their interaction studies with calf thymus DNA, 2005, vol. 30, position 8, pp.998-1007, the abstract, [on-line] [found on 29.06.2009]. Found on the Internet <URL://http://www.springerlink.com/content/p7n716022572p366/fulltext.pdf?page=l> | 1-25 |
| | ./.. | |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 29 June 2009 | 09 July 2009 |
| Name and mailing address of the ISA/ RU | Authorized officer |
| Facsimile No. | Telephone No. |

Form PCT/ISA/210 (second sheet) (April 2005)

**INTERNATIONAL SEARCH REPORT**

International application No.
PCT/RU2009/000070

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2007/073134 A1 (ESPINOSA ABDALA, LEOPOLDO DE JESUS) 28.06.2007, the abstract, page 9, lines 13-19, claim 1 | 1-25 |
| A | EP 0256645 A2 (ALBION INTERNATIONAL, INC.) 24.02.1988, the abstract, page 3, lines 10-11, 14-15, page 6, lines 5-10 | 1-25 |
| A | MARINO TIZIANA et al. Structural and Electronic Characterization of the Complexes Obtained by the Interaction between Bare and Hydrated First-Row Transition-MetalIons (Mn2+, Fe2+, Co2+, Ni2+, Cu2+, Zn2+) and Glycine. Joumal of Physical Chemistry. B, 2006, vol. 110, position 48, pp. 24666-24673 | 1-25 |

Form PCT/ISA/210 (continuation of second sheet) (April 2005)

INTERNATIONAL SEARCH REPORT

**continuation sheet**

International application No.
PCT/RU 2009/000070

A. CLASSIFICATION OF SUBJECT MATTER

***A61K 9/08*** *(2006.01)*
***A61K 33/24*** *(2006.01)*
***A61K 31/198*** *(2006.01)*
***A61K 38/00*** *(2006.01)*
***A61P 37/00*** *(2006.01)*
***A61P 43/00*** *(2006.01)*

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Basolo F. ; Pearson R.** Mechanisms of inorganic reactions. *Investigation of complexes of metals in solution,* 1971, 592
- **Tolstikova T.G. et al.** *On the road to low-dose medicines* / *Vestnik rossiiskoi akademii nauk,* 2007, vol. 77 (10), 867-874
- **Alonso-Torre S.R. ; Trautmann A.** Calcium responses elicited by nucleotides in macrophages. Interaction between two receptor subtypes. *J. Biol. Chem.,* 1993, vol. 268, 18640-18647
- Basic & Clinical Pharmacology. 1995
- Induction and collection of peritoneal exudate macrophages. **Conrad R.E.** Manual of macrophages methodology. Marcell Dekker, 1981, 5-11
- **Grynkiewicz G. ; Poenie M. ; Tsien R.Y.** A new generation of Ca2+ indicators with greatly improved fluorescence properties. *J. Biol. Chem.,* 1985, vol. 260, 3440-3450
- **Monahan R.A. ; Dvorak H.F. ; Dvorak A.M.** Ultrastructural localization of nonspecific esterase activity in guinea pig and human monocytes, macrophages and lymphocytes. *Blood,* 1981, vol. 58, 1089-1099
- **Pirmohamed M. ; Kevin B.** Genetic susceptibility to adverse drug reactions. *Trends Pharm. Sci.,* 2001, vol. 22, 298-305
- **Randriamampita C. ; Trautmann A.** Ion channels in murine macrophages. *Cell Biol.,* 1987, vol. 105, 761-769
- **Spiccia L.** *Inorg. Chim. Acta.,* 2004, vol. 357, 2799
- **Junk P.C. ; McCool B.J. ; Moubaraki B. et al.** *Angewandte Chemie. Int. Ed. Engl.,* 1999, vol. 38, 2224